# EUROPÄISCHE PATENTSCHRIFT

(11) **EP 2 018 835 B1**
(45) Veröffentlichungstag und Bekanntmachung des Hinweises auf die Patenterteilung: **05.03.2014**
(21) Anmeldenummer: 08011985.2
(22) Anmeldetag: 03.07.2008
(51) Int. Cl.: A61F 13/00

(54) **Wirkstoff abgebendes Pflaster**
Agent-eluting plaster
Pavé sortant d'agent actif

(30) Priorität: 09.07.2007 EP 07013361
(43) Veröffentlichungstag der Anmeldung: 28.01.2009
(73) Patentinhaber: Bader, Augustinus, 04668 Parthenstein / OT Klinga (DE)
(72) Erfinder: Bader, Augustinus, 04668 Parthenstein / OT Klinga (DE)
(74) Vertreter: Benz, Jürgen

(56) Entgegenhaltungen:
- EP-A1- 0 334 306
- WO-A1-94/15562
- WO-A2-2007/033678
- US-A- 4 990 144
- US-A1- 2006 041 247

## Beschreibung

### TECHNISCHES GEBIET DER ERFINDUNG:

Die Erfindung betrifft ein medizinisches Pflaster, welches normale und verletzte Haut oder Schleimhaut, insbesondere offene Wunden mit einem oder mehreren Wirkstoffen versorgen kann, die dazu beitragen, dass die Regeneration der Haut oder die Wundheilung beschleunigt und verbessert von statten geht. Weiterhin kann das System auch zur Versorgung von Wirkstoffen für den Gesamtorganismus verwendet werden, wobei die Dosierung nicht wie bisher üblich über Konzentrationsveränderungen des Wirkstoffes erreicht wird.

Das Pflaster weist dabei neuartige konstruktive Merkmale auf, die es ermöglichen, dass neben konventionellen chemischen Stoffen wie z.B. Schmerzmittel, Nikotin, insbesondere protein- oder peptidhaltige Arzneimittel ihre regenerative, insbesondere Wunden heilende oder die Wundheilung promovierende Wirkung durch Abgabe aus dem Pflaster ausüben können, ohne dass sie dabei durch im Wundsekret vorkommende Substanzen wesentlich inaktiviert oder metabolisiert werden.

Überdies kann der Aufbau des Pflasters so gestaltet sein, dass ein Wirkstoff über einen längeren Zeitraum hinweg, auf einen bestimmten Patienten individuell angepasst, gleichmäßig an die Wunde bzw. die Haut abgegeben werden kann, ohne dass das Pflaster gewechselt werden muss.

Die erfindungsgemäßen Pflaster sind insbesondere für die Abgabe von Proteinen als Wirkstoff mit verhältnismäßig kurzer Halbwertszeit, beispielsweise Erythropoietin (EPO) in all seinen bekannten Varianten und Formen, geeignet. Im Falle von EPO ist man dabei an dessen Gewebe schützenden oder regenerativ wirkenden Anwendungen interessiert unter Umgehung des Problems der Verabreichung von Erythropoietin mit seiner hier nicht erwünschten, die Blutbildung fördernden Wirkung.

### TECHNISCHER HINTERGRUND DER ERFINDUNG

Wundheilung z.B. von verletzter Haut oder Schleimhaut läuft gewöhnlich in drei Phasen ab: die Entzündungsphase, die Proliferationsphase und die Wiederaufbau/Remodelling-Phase. Bei einer frischen Wunde oder Hautverletzung, die es zu versorgen gilt, spielen sich innerhalb der ersten 24 Stunden inflammatorische Prozesse ab, welche vor allem das Einwandern diverser Entzündungsfaktoren (wie z.B. Fibronectin) und verschiedenartiger Zellen, wie beispielsweise Monozyten, Phagozyten, polymorphe Zellen und Makrophagen umfassen und schließlich zur Ausbildung einer Fibrinmatrix und vaskulärer Endothelzellen führt. Das dabei entstehende Wundsekret enthält neben den genannten Faktoren und Zellen auch Zelltrümmer und eine Reihe von proteolytischen Enzymen sowie in die Wunde eingedrungene Bakterien, die diesbezüglich wirkende Stoffe enthalten.

Die zum Teil hoch aktiven proteolytischen Enzyme sind der Grund, warum auf die Wunde aufgebrachte, die Wundheilung fördernde protein- oder peptidhaltige Arzneimittel oft wenig oder gar nicht wirksam sind, da das betreffende Protein oder Polypeptid aufgrund seiner chemischen und biologischen Natur durch besagte Enzyme inaktiviert, gespalten und abgebaut werden, ehe sie eine ausreichende pharmakologische Wirksamkeit entfalten können. Durch Infizieren der Wunde mit Bakterien oder Einwandern von Zelldebris wird das Problem zusätzlich verstärkt. Bei all diesen Vorgängen spielt die jeweilige Halbwertszeit des biologischen Abbaus des betreffenden Arzneimittel-Proteins unter den gegebenen physiologischen Bedingungen eine entscheidende Rolle bei der Frage, ob das besagte Protein eine ausreichende bzw. ausreichend lange Wirksamkeit bei Verletzungen der Haut, Schleimhaut und dergleichen besitzt, sofern es nicht systemisch, wie beispielsweise subkutane oder intravenöse Applikation sondern durch Auftragen auf die Wunde, also topisch, geschieht.

Aus diesem Grund sind bislang insbesondere gegenüber enzymatischen Prozessen empfindliche Proteine und Polypeptide für topische Anwendung bei Hautwunden nicht oder nicht ausreichend erfolgreich eingesetzt worden, selbst dann, wenn versucht wurde, eine physikalische Barriere zwischen dem Wirkstoff und der Wunde einzuführen und diese möglichst lange aufrecht zu erhalten, beispielsweise durch entsprechende poröse Membranen, die nur Moleküle unter 50.000 Dalton passieren lassen. Die meisten proteolytische Enzyme weisen jedoch eine Größe über 100.000 Dalton, was sie daran hindert in das Wirkstoffdepot oberhalb der Wunde einzuwandern. Aber auch unter dieser Maßnahme sind die effektiv wirksamen Halbwertszeiten von Proteinwirkstoffen sehr gering, da sie bei Eintritt in die Wunde durch proteolytische Enzyme im sich bildenden Wundsekret schnell gespalten und in der Regel dadurch pharmakologisch unwirksam gemacht werden.

Eine andere Möglichkeit dem vorzeitigen Abbau des Wirkstoff-Proteins entgegen zu wirken, wird zuweilen in der Bereitstellung von "Slow Release" - Formulierungen gesehen, bei denen der Wirkstoff in ein biologisch abbaubares Polymermaterial eingebracht wird, aus dem er entsprechend der Kinetik des Abbaus des Polymers freigesetzt wird. Diese Kinetik entspricht aber oft nicht der Kinetik des Abbaus des Wirkstoffes nach Freisetzung in die Wunde.

Nicht zuletzt aus diesem Grund werden pharmazeutische Proteine in der Regel systemisch appliziert, wodurch ihre Halbwertszeit beträchtlich verlängert werden kann und sie auch schneller an die Stellen im Körper transportiert werden, an denen sie ihre therapeutische Wirksamkeit entfalten sollen. Allerdings müssen bei dieser Applikationsmethode die Dosen des proteinhaltigen Wirkstoffes ausreichend hoch sein um den gewünschten therapeutischen Effekt zu erzielen, was oft zwangsläufig zu unerwünschten Nebenreaktionen führt.

Im Falle der therapeutischen Behandlung von Hautverletzungen erscheint überdies eine systemische Applikation eines Wirkstoffes prinzipiell weniger angebracht, da die heilende Wirkung des Arzneimittels eigentlich nur lokal erforderlich ist. Somit besteht ein generelles Problem, wenn zur Behandlung von Hautverletzungen und offenen Fleisch- und Hautwunden proteinhaltige Wirkstoffe eingesetzt werden sollen.

Proteinwirkstoffe zum Einsatz bei derartigen Verletzungen, wie sie bei übermäßigen mechanischen Einwirkungen und Irritationen sowie bei Verbrennungen und Verbrühungen auftreten können, sind prinzipiell bekannt. So wird in jüngerer Zeit der Einsatz von Wachstumsfaktoren, wie beispielsweise EGF, TGF beta, GCSF, GM-CSF, HGH, CNTF, EPO oder TPO bei der Heilung von derartigen Zuständen diskutiert.

Insbesondere der noch nicht sehr lange bekannten nicht-hämatopoietischen Wirkung von Erythropoietin (EPO) im Zusammenhang beispielsweise mit der angeregten Bildung und Regeneration von Endothel- und Gewebezellen, wie Bindegewebe, Muskelgewebe, Epithelgewebe und Nervengewebe, wird zunehmend Bedeutung beigemessen.

So beschreibt die WO 2004/001023 unter anderen die Verwendung von EPO und TPO zur Anregung der Gefäßneubildung und Geweberegeneration und Verbesserung der Wundheilung, z.B. nach Operationen oder Verletzungen.

In der WO 2005/063965 wird die Verwendung von EPO zur gezielten strukturell gelenkten Regeneration von traumatisiertem Gewebe gelehrt, wobei auch topische bzw. transdermale Applikation des Wirkstoffes vorgeschlagen werden, bei welcher nicht nur ein Endothelzellwachstum stimuliert, sondern auch die Parenchymregeneration und die Ausbildung der Wandstrukturen gefördert wird, so dass ein koordiniertes dreidimensionalen Wachstum zum Aufbau eines funktionsfähigen Gewebes, Organs oder Teilen davon von statten geht.

Die WO 2005/070450 beschreibt die Verwendung von EPO bei der Regeneration von Gefäßen und Gewebe mit eine wöchentlichen Dosis von unter 90 IU/kg Körpergewicht auch für den Bereich der Wundversorgung. Auch wenn hierbei prinzipiell von der möglichen topischen Anwendung gesprochen wird, so wird dennoch betont, dass die systemische Applikation bevorzugt wird.

Haroon et el. (American J. Pathol. 2003, 163, 993) diskutieren die neue Rolle von EPO im Rahmen der durch Fibrin induzierten Wundheilungsprozesse.

In einem Review-Artikel diskutieren Brines und Cerami (Kidney International, 2006) die Rolle von EPO bei der Protektion von Gewebe.

Erythropoietin, EPO-Derivate, wie z.B. pegyliertes oder dimerisiertes EPO (z.B. WO 02/49673 oder WO 01/02017), sowie vermutlich auch entsprechend wirksame synthetische EPO Peptid-Mimetika (wie bekannt, z. B. aus WO 96/40749, WO 96/40772, WO 01/38342, WO 01 091780, WO 2004/101611; WO 2004/100997, WO 2004/101600, WO 2004/ 101606 und WO 2006/050959) scheinen also hervorragend geeignet zu sein, um bei Verletzungen der Haut, der Schleimhaut, bei offenen Haut- und Fleischwunden oder auch bei Hautirritationen durch Verbrennungen oder Verbrühungen die Neubildung und Regeneration des betroffenen Gewebes gezielt zu initiieren und zu steuern und letztlich die Heilung fördern und beschleunigen zu können.

Es wäre somit wünschenswert, EPO, EPO-Derivate, EPO-Peptidmimetika und andere ähnlich oder anders wirkende Protein- oder Peptid-Wirkstoffe für diese Anwendungen in Form eines topisch zu applizierenden Präparats zur Verfügung zu stellen. Da die Halbwertszeit von EPO im Plasma nur etwa 48 Stunden beträgt, ergibt sich in der Regel eine unzureichende oder zumindest nicht zufrieden stellende Wirkung bei topischer Anwendung, die auch durch Pegylierung oder Dimerisierung des Moleküls und der damit bedingte Verlängerung der Plasma-Halbwertszeiten nicht wesentlich verbessert werden kann.

Es besteht somit die Aufgabe insbesondere EPO und seine bioäquivalenten Derivate, Fragmente, Mimetika und dergleichen, aber auch andere für die Wundheilung geeignete bzw. wirksame Proteine oder Peptide für die topische Anwendung bei den genannten Wundindikationen bereit zu stellen, ohne dass es zu dramatischen Wirkungsverlusten durch Proteolyse auf Grund enzymatischer oder anderer Vorgänge in der Wunde kommt.

### BESCHREIBUNG DER ERFINDUNG:

Die gestellte Aufgabe kann überraschenderweise durch ein spezielles neuartiges den betreffenden Wirkstoff enthaltendes Pflaster gelöst werden, welches durch sein neuartiges Design und seine technische Ausstattung eine proteolytische Spaltung des Wirkstoff-Proteins zu verringern oder sogar zu verhindern vermag, sowie die kontrollierbare regionalisierte therapeutische Wirkung von Wirkstoffen wie EPO erreichen kann, um dadurch direkt ortständige Stamm- und Vorläuferzellen zu stimulieren.

Weiterhin kann das System auch zur Versorgung von Wirkstoffen für den Gesamtorganismus verwendet werden, wobei die Dosierung nicht wie bisher üblich über Konzentrationsveränderungen des Wirkstoffes erreicht werden kann.

Das Ziel ist es, eine erhöhte Sicherheit trotz Verbesserungen in der Anwenderfreundlichkeit zu erhalten. Die Schwierigkeiten in der üblichen Vorgehensweise ist, dass Über- und Unterdosierungen durch Konzentrations-veränderungen auftreten können, die für den Anwender nicht nachvollziehbar sind. Eine optische Überprüfung für den Endanwender ist nicht möglich. Die Gefahren der systemischen Gabe werden hierdurch reduziert und die therapeutische Breite in Bezug auf die systemischen Nebenwirkungen reduziert. Vor der generischen Aufnahme wird insbesondere das therapeutische Protein durch regionale Proteasen abgebaut. Dies entspricht der Barrierefunkition der Haut.

Zur Unterstützung der gewebeprotektiven oder regenerativ wirkenden Anwendung, gab es daher Überlegungen subpolycythämische Konzentration unterhalb von 90 U/Kg KGW einzusetzen (WO 2005/070450). Diese Konzentration hat jedoch den Nachteil, dass hier nur schwache regenerative Effekte entstehen, welche weitestgehend auf die Endothelzellbildung begrenzt sind. Hohe Konzentration vor Ort, die zur Stimulierung der betaCR- Untereinheit des EPO Rezeptors beitragen, werden nur ungenügend erreicht. Der Grund liegt darin, dass es sich nicht um Zellen handelt, die wie Endothelzellvorläuferzellen im Blut zirkulieren. Vorläuferzellen der Haut liegen in den dermalen Anteilen oder auch Kryptenstrukturen der Dermis vor. Diese können durch eine topische Applikation sehr viel direkter und vor allem unabhängig von den schwierig einzustellenden und nebenwirkungsreichen systemischen Applikationsgrenzen angesprochen werden. Zusätzlich wird dadurch eine höhere therapeutische Breite erreicht, da die Prozessierung und der Abbau des EPO auch bereits vor Ort in der Wunde durch Proteasen erreicht werden kann.

Der duale Vorteil der Einstellbarkeit höherer regionaler Wirkstoffkonzentration am Wirkort und die verbesserte therapeutische Breite für den Gesamtorganismus in Bezug auf Konzentration, die als IU/Kg Körpergewicht errechnet werden müssen, wird in Kombination mit dem erfindungsgemäßen Pflaster noch mit dem Vorteil der Wundabdeckung, der Feuchtigkeitskontrolle als auch der über die Größe des Pflasters mögliche Dosierungsfähigkeit ergänzt.

Wundpflaster, die einen Wirkstoff enthalten und diesen an die Wunde abgeben zu vermögen, sind prinzipiell bekannt.

So ist in der WO 03/101361 ein Wirkstoff-Abgabesystem in Form eines Pflasters beschrieben, bei dem in einer Tasche, die von einer oberen undurchlässigen und unteren permeablen Schicht des Pflasters gebildet wird, ein fließfähiger Träger mit einem medizinischen Wirkstoff vorliegt, wobei letzterer an die Wunde abgegeben wird.

Die WO 2006/109325 beschreibt ein im Prinzip ähnliches Pflaster, bei dem ein in flüssiger oder fließfähiger Wirkstoff in einem kleinen Behälter auf der Oberseite des Pflasters vorliegt. Der Behälter kann gezielt geöffnet werden, wodurch der Wirkstoff auf eine darunter befindliche Schicht gelangt und von dieser aufgesaugt wird. Diese Schicht gibt dann den Wirkstoff an die darunter liegende Wunde ab.

Beide Pflaster lösen das oben beschriebene Problem für proteinhaltige Wirkstoffe nicht.

Die vorliegende Erfindung basiert auf der Erkenntnis, dass durch die simultane Freisetzung von Wirkstoff-Protein aus dem Pflaster und Entfernung oder Abtrennung von Proteolyse förderndem Wundsekret, welches sich auf / in der Wunde unterhalb des die Wunde abdeckenden Pflasters kontinuierlich während des Heilungsprozesses bildet, die Halbwertszeit der Wirksamkeit des Proteins auf bzw. in der Wunde auch in kleinsten Bereichen des Pflasters beträchtlich erhöht wird und Werte erreicht, welcher mindestens der Halbwertszeit des selben Proteins, die im Plasma erreicht werden könnte, entspricht, oft sogar diese Werte übertrifft.

Damit dies geschieht, ist das erfindungsgemäße Pflaster in Mikrodomänen ähnlicher oder identischer Geometrie aufgeteilt, wobei die Mikrodomänen im wesentlichen ein oder in der Regel mehrere in der Trägermatrix des Pflasters befindliche abgeschlossene Kavitäten umfassen, welche Minidepots oder Cluster von Minidepots für den Proteinwirkstoff darstellen, und eine oder mehrere offene Kavitäten umfassen, welche in der Regel Kanäle oder Kanäle mit lokalen Ausweitungen sind, die miteinander verbunden sind, und welche die geschlossenen Kavitäten (Behältnisse/Depots) oder deren Cluster umgeben. Erfindungsgemäß grenzt eine Depotstruktur an eine Kanalstruktur und umgekehrt.

Die "offenen" Kavitäten, bzw. Kanalstrukturen, haben die Aufgabe das Wundsekret aus der Umgebung der Mikrodomäne bzw. des Minidepots oder der Cluster von Minidepots zu entfernen und vorzugsweise zu größeren oder Haupt-Kavitäten im Pflaster zu transportieren, von wo aus es aus dem Pflaster entfernt werden kann. Das Wundsekret kann dabei mittels entsprechender Anschlüsse, die vorzugsweise an den Hauptkavitäten angebracht sind aktiv abgesaugt werden (in einfachsten Fall durch eine Spritze) oder passiv von Drainagen aufgenommen werden, die vorzugsweise in die Kavitäten, insbesondere Hauptkavitäten eingebracht werden und von dort eine Kapillarwirkung entfalten.

Durch diese konstruktive Maßnahmen unterliegt das aus den Depots sukzessive an die Wunde abgegebenes Wirkstoff-Protein überraschenderweise nicht oder nur in geringem Maß der proteolytischen Aktivität der Wundsekretenzyme, so dass eine ausreichende Wirkung im Sinne der oben angesprochenen Regeneration von Gefäß- und vor allem von Gewebestrukturen über einen längeren Zeitraum beobachtet wird.

Im Falle von EPO ist es so völlig ausreichend, das Pflaster abhängig von der eingesetzten EPO Menge alle 48 - 72 Stunden zu wechseln, um den gewünschten Effekt zu erzielen, während bei topischer Gabe mittels eines Pflasters des Standes der Technik oder durch direktes Einbringen in / auf die Wunde z.B. mittels eines Gels oder einer Salbe die besagte nichthämatopoietische Wirkung nicht oder nur in unbefriedigender Weise eintritt.

Innerhalb der genannten 48 - 72 Stunden (bei anderen Protein-Wirkstoffen kann der Zeitraum kürzer oder länger sein), kann oder sollte das Wundsekret z.B. durch Absaugen mittels der vorgesehenen Anschlüsse immer wieder abgeführt werden. Falls dies nicht oder schlecht möglich ist, wie beispielsweise bei Wunden in der Mundhöhle oder im Kieferbereich (z.B. bei Unfallchirurgie, Kieferoperationen oder Zahntransplantationen, Zahnextraktionen) kann der gleiche Effekt erreicht werden, wenn die kanalähnlichen Domänen oder Strukturen ersatzweise Drainagematerial enthalten, das kontinuierlich in der Mikroumgebung eines jeden Depots oder Minibehälters, der den Protein-Wirkstoff enthält, Wundsekret und damit proteolytische Enzyme aufnimmt und von dem Protein-Wirkstoff abtrennt.

Die Kavitäten bzw. Kanäle in der Mikroumgebung eines jeden Depots in der Pflasterstruktur dienen erfindungsgemäß aber nicht nur dem Transport oder der Aufnahme von Wundsekret sondern auch der Zufuhr von Luft und / oder anderen Wirkstoffen und / oder Zellen, oder Zellbestandteile, welche die Heilung insgesamt fördern. Die Zufuhr derartiger Hilfsmittel erfolgt erfindungsgemäß je nach Bedarf und individuell über einen oder vorzugsweise mehrere Anschlüsse oder Öffnungen ("Ports"), die in gewissen, vorzugsweise regelmäßigen Abständen entlang der kanalähnlichen Struktur, vorzugsweise an Kreuzungspunkten oder Aufweitungen, angebracht sind.

Solche anderen Wirkstoffe können andere Wachstumsfaktoren, Desinfektionsagenzien, Antibiotika, Proteinaseinhibitoren, Kollagen und dergleichen sein, welche durch die genannten Anschlüsse in die Pflasterstruktur eingebracht werden können.

Auch ist es erfindungsgemäß möglich, über diese Anschlüsse bei Bedarf vorzugsweise autologe Zellen, wie Fibroblasten, Endothelzellen, Makrophagen, Monozyten, Kollagen-/ Elastinfaserzellen oder auch Stammzellen in die Wunde einzubringen, welche die Gewebe- und Gefäßregeneration und damit die Wundheilung promovieren und beschleunigen.

Die Trägerstruktur des Pflasters umfasst also abgeschlossene Kavitäten, die als Behältnisse für das Arzneimittel dienen. Sie umfasst aber auch offen Kavitäten, zumeist in Form von Kanälen, welche mit einander in Verbindung stehen und die Behältnisse oder Cluster von Behältnissen umgeben und zwar in dem Abstand, dass ein kontinuierlicher Abtransport des Wundsekrets über die Kanäle gewährleistet ist.

Gegenstand der Erfindung ist somit ein Pflaster zur Versorgung von normaler und / oder verletzter Haut, Schleimhaut oder offenen Wunden mit einem Wirkstoff oder Arzneimittel, umfassend eine Trägermatrix, welche den Wirkstoff oder das Arzneimittel enthält, wobei die Trägermatrix Bereiche (i) in Form einer oder mehrerer als Behältnis oder Depot für das Arzneimittel dienenden Strukturen und Bereiche (ii) in Form von einer oder mehreren Kavitäten umfasst, welche der Aufnahme und Abführung von Wundsekret und / oder Belüftung und / oder der topischen Einbringung von weiteren Wirkstoffen und / oder von die Heilung der Haut, Schleimhaut oder offenen Wunde fördernde Zellen dienen, wobei
(a) mindestens ein Bereich gemäß (i) an mindestens einen Bereich gemäß (ii) grenzt,
(b) die Bereiche (i) und (ii) auf der von der Wunde abgewandten Seite verschlossen sind, so dass besagte Arzneimittel, besagte weitere Wirkstoffen, besagte Zellen sowie Wundsekret abgegrenzt sind und auch nicht die abgrenzende Schicht durchdringen können,
(c) die Bereiche (i) und (ii) auf der der Wunde zugewandten Seite offen oder für besagte Arzneimittel, Wundsekret und weitere Wirkstoffe / besagte Zellen zumindest passierbar sind,
(d) die als Kavitäten ausgebildeten Bereiche (ii) in der Ebene des Pflasterfläche in Form von Kanälen, kanalähnlichen Strukturen und Hohlraum ähnlichen Erweiterungen der Kanalstrukturen angeordnet sind, und
(e) die als Kavitäten ausgebildeten Bereiche (ii) mindestens einen Anschluss/Öffnung/Port für eine Spritze oder Absaug- / Zuführungsvorrichtung aufweisen, der es ermöglicht, entweder unter Erzeugung eines im Pflaster erzeugten Unterdrucks in den Kavitäten angesammeltes Wundsekret abzuführen und /oder gegebenenfalls besagte weitere Wirkstoffe zu applizieren.

Als Wunden der Haut oder Schleimhaut werden erfindungsgemäß alle solche angesehen, die durch mechanische, thermische, chemische Einflüsse, durch Strahlung oder durch krankheitsbedingte und / oder inflammatorische Prozesse (z.B. Ekzeme, Abszesse, Allergien, Ausschläge aller Art, etc.) entstanden sind.

Im Falle stark nässender oder viel Wundsekret produzierender Haut oder Wunden können die Kavitäten der Bereiche (ii) zusätzlich Drainagemittel aufweisen, die es ebenfalls ermöglichen, in den Kavitäten angesammeltes Wundsekret oder Feuchtigkeit abzuführen. Im Falle von mehr trockenen Verletzungen, z. B. bei Sonnenbrand oder leichteren Brandtverletzungen kann auch ein Pflaster eingesetzt werden, welches ohne zusätzliche Drainagemittel ausgestattet ist.

Erfindungsgemäß sind die als Behältnis bzw. als Depot für das Arzneimittel dienen Strukturen der Bereiche (i) wannenförmig, wobei die offenen Seite der Wannen zur Wunde hin ausgerichtet ist, und die Wannen von beliebiger, irregulärer, vorzugsweise aber regulärer, insbesondere rechteckiger, quadratischer, sechseckiger / wabenförmiger oder runder Grundfläche sind. Vorzugsweise weisen sie eine sechseckige/wabenförmige Grundflächenform auf.

Ein einzelnes Minidepot oder Behältnis der Bereiche (i) besitzt erfindungsgemäße eine lichte Tiefe oder Dicke in der Trägerstruktur des Pflasters von 0.2 bis 5mm, vorzugsweise von 0.3 bis 3mm und insbesondere von 0.3 bis 2.0 mm. Die besagte Dicke oder Tiefe eines einzelnen solchen Minibehälters richtet sich nach der gewünschten zu erzielenden Depotwirkung des Wirkstoffes: dickere Pflastere können mehr Wirkstoff aufnehmen und die Abgabe an die Wunde erfolgt länger. Abweichungen der Dimensionen nach unten in den Nanobereich als auch nach oben in den Makrobereich sind in Bezug auf die Anwendungs- und Materialanforderungen jederzeit möglich.

Der lichte Durchmesser eines einzelnen Behältnisses bzw. einer einzelnen Kammer in der Trägermatrix des Pflasters kann zwischen 0.2 und 5mm, vorzugsweise zwischen 0.5 und 5mm, insbesondere zwischen 1.0 und 3mm betragen, und bestimmt somit die Fläche mit der das Wirkstoffdepot Kontakt hat mit einem Teil der Wunde. Überraschenderweise hat sich gezeigt, dass bei insbesondere einem lichten Durchmesser eines Behältnisses zwischen 1 und 3mm die Wirksamkeit des an die Wunde abgegebenen bzw. freigesetzten Protein-Wirkstoffes höher ist als bei Kavitäten mit deutlich größerer Kontaktfläche zur Wunde.

Wie bereits erwähnt, können die als Behältnis bzw. Depot für das Arzneimittel dienenden Kavitäten zu Clustern von 2 bis 200 Behältnissen zusammengefasst sein, welche durch dünne Stege voneinander getrennt sind. Dabei können die Clusterstrukturen, wie schon auch die durch sie gebildeten einzelnen Depotstrukturen verschiedenartige geometrische Grundflächenformen aufweisen. So können die letzteren beispielsweise rechteckig, quadratisch, wabenförmig (sechseckig) oder rund sein.

Die Stege zwischen den Behältnissen sind zweckmäßigerweise aus dem Material der Trägermatrix und weisen eine Dicke zwischen 0.1 und 3mm auf, vorzugsweise zwischen 0.1 und 0.5mm. Die Stege zwischen den wannenförmigen Behältnissen bzw. Minidepots können erfindungsgemäß selbst kanalähnliche Strukturen aufweisen (beispielsweise in Form eine Hohlprofils), welche der Abführung vom Wundsekret, und oder der Belüftung und oder der Zuführung von weiteren Wirkstoffen / besagte Zellen dienen. Stege, die ein Hohlprofil aufweisen, weisen eine Dicke vorzugsweise zwischen 0.5 und 3mm auf und sind insbesondere dort angebracht, wo einzelne Depots zu größeren Clustern zusammengefasst wurden, und die Fläche mit der ein Cluster die Wunde abdeckt, so groß ist, dass die die Clusterdomäne umgebende Kanalstruktur nicht ausreicht, um das unter der in diesem Fall großen Clusterdomäne sich ansammelnde Wundsekret abtransportieren zu können.

Aus diesem Grund sollte ein Cluster einen mittleren Durchmesser besitzen, der nicht wesentlich größer als 25mm sein sollte und vorzugsweise zwischen 5 und 25mm liegt, je nach dem wie groß die einzelnen Behältnisse des Clusters gewählt wurden. Eine entsprechende Clusterstruktur kann zwischen 10 und 200, vorzugsweise zwischen 25 und 100 Minidepots bzw. Einzelstrukturen der Bereiche (i) aufweisen.

Es hat sich als vorteilhaft gezeigt, wenn die gesamte Fläche, welche die kanalähnlichen Kavitäten der Bereiche (ii) auf dem Pflaster einnimmt > 1 %, > 5%, > 10%, >20%, > 30% oder >40% der Fläche ist, welche von den Depotdomänen der Bereiche (i) eingenommen wird.

Vorzugsweise entspricht die Fläche der Kanalstrukturen 2 - 30% , vorzugsweise 5 - 20% der Fläche der Depotstrukturen. Letztlich hängt das Verhältnis von der Art und Menge der sekretierenden Gewebs- und Wundflüssigkeiten ab. Bei stark nässenden bzw. sekretierenden Wunden sollten die kanalähnlichen Strukturen der Bereiche (ii) etwa 20 bis 40% der wirksamen Gesamtfläche des Pflasters einnehmen, während bei nur wenig Wundsekret produzierenden Wunden ein entsprechender Anteil von 2 - 10 % ausreichend ist.

Gegenstand der Erfindung ist somit ein entsprechendes Pflaster, bei dem die Dicke der arzneimittelhaltigen Strukturen (i) 0.2 bis 5.0mm, vorzugsweise 0.3 bis 2.0 mm beträgt. Gegenstand der Erfindung ist ferner ein entsprechendes Pflaster, bei dem eine einzelne Struktur der Bereiche (i) einen mittleren Durchmesser bzw. Abstand von ihren gegenüber liegenden Kanten von 0.5 bis 5.0 mm aufweist.

Gegenstand der Erfindung ist ferner ein entsprechendes Pflaster, bei dem die einzelnen Strukturen der Bereiche (i) in Clustern zusammengefasst sind, welche einen mittleren Durchmesser bzw. Abstand von ihren gegenüber liegenden Kanten von 5.0 bis 25.0 mm, vorzugsweise 10.0 bis 15.0mm besitzen.

Gegenstand der Erfindung ist auch ein entsprechendes Pflaster, bei dem die einzelnen das Arzneimittel enthaltenden Strukturen der Bereiche (i) in Clustern zusammengefasst sind, wobei jeder Cluster vorzugsweise bis zu 200, insbesondere 25 bis 100 wannenähnliche Einzelstrukturen der Bereiche (i) aufweist.

Die Kanäle bzw. kanalähnlichen Strukturen der Bereiche (ii), welche generell ein Hohlprofil mit einer offen Seite zur Wunde besitzen, weisen eine mittleren lichten Durchmesser von 0.3 bis 3.0 mm auf.. Dabei kann ein erfindungsgemäßes Pflaster Kanäle von unterschiedlicher Dicke bzw. Durchmesser besitzen in Form von Haupt- und Nebenkanälen. Insbesondere die Hauptkanäle können in regelmäßigen oder unregelmäßigen Abständen über einen kleinen Bereich eine größeren Durchmesser besitzen, wodurch in diesem Bereich ein kleiner Hohlraum in Form eine Blase, Kugel oder Kalotte mit einem Durchmesser vorliegt, der größer ist als der Kanal selbst.

Solche Aufweitungen oder Verdickungen sind vorzugsweise im Kreuzungsbereich zweier sich schneidender bzw. treffender Kanäle vorgesehen, können aber auch davon unabhängig entlang eines Kanals vorgesehen werden. Im Kreuzungsbereich zweier sich schneidender Kanäle bewirken diese Aufweitungen ein rascheres und kontinuierliches Zu- oder Ableiten von Wundsekret oder der oben genannten anderen Wirkstoffe, Zellen und anderer Hilfsstoffe.

Daher ist es vorteilhaft, diese Aufweitungsbereiche zumindest teilweise mit Anschlüssen/Öffnungen/Ports zum Absaugen, vorzugsweise durch entsprechend gestaltete Spritzen, oder zur Zuführung der genannten Stoffe, oder zur Belüftung der Wunde auszustatten.

Im erfindungsgemäßen Pflaster wechseln Bereiche oder Domänen der Struktur (i) (Wirkstoffdepots bzw. Cluster von Wirkstoffdepots) mit Bereichen oder Domänen der Struktur (ii) (Kanäle) ab, wobei, wie bereits erwähnt, zu große Gebiete ohne Kanäle vermieden werden müssen, um nicht den Wirkstoffeffekt durch zuviel vorhandenes Wundsekret zu verringern. So sollte der Abstand zwischen zwei kanalähnlichen Strukturen der Bereiche (ii) und damit auch der Abstand zweier Depotstrukturen (i) bzw. Clusterstrukturen zwischen 5 und maximal 25mm, vorzugsweise zwischen 5 und 15mm, liegen.

Gegenstand der Erfindung ist somit auch ein entsprechendes Pflaster bei dem die kanalähnlichen Strukturen (ii) einen mittleren Durchmesser von 0.3 bis 3.0 mm aufweisen, wobei Hauptkanäle zwischen 1.0 und 3.0mm und Nebenkanäle zwischen 0.3 und 1.0mm dick sind.

Gegenstand der Erfindung ist auch ein entsprechendes Pflaster bei dem der Abstand zwischen zwei kanalähnlichen Kavitäten der bereiche bzw. Domänen (ii) maximal 25mm, vorzugsweise 5 bis 25 mm beträgt.

Gegenstand der Erfindung ist ferner ein Pflaster, welches sich schneidende kanalähnliche Kavitäten der Bereiche/Domänen (ii) aufweist und der Anschluss für eine Absaug-/ Zuführungsvorrichtung im Kreuzungsbereich zweier solcher Kanalstrukturen der Bereiche (ii) angebracht ist. Vorzugsweise sind zumindest einiger dieser Kreuzungsbereiche hohlraumartig erweitert, um mehr Platz für angesammeltes Wundsekret und die Absaugvorrichtung zur Verfügung zu stellen, welche im einfachsten Fall ein Anschlussstutzen oder eine Öffnung für eine Spritze oder Absaugpumpe ist.

Gegenstand der Erfindung ist in diesem Sinne daher auch ein Pflaster, welches sich schneidende kanalähnliche Strukturen der Bereiche (ii) aufweist und einer oder mehrere der entstandenen Kreuzungsbereiche kalotten-, blasen- oder kugelförmig vergrößert ist.

Gegenstand der Erfindung ist ferner ein Pflaster, welches Kavitäten bzw. kanalähnliche Strukturen der Bereiche (ii) umfasst, die sich nicht schneiden oder kreuzen, sondern ring- oder spiral- oder meanderförmig angeordnet sind und sich mit Ringen, Spiralen oder Meandern abwechseln bzw. an diese angrenzen, wobei zwei gleichartige Domänen einen mittleren Abstand zueinander von maximal 25mm, vorzugsweise 5 bis 25 mm aufweisen.

Die Anordnungen der Kavitäten (i) und (ii) auf dem erfindungsgemäßen Pflaster können in ihrer Geometrie und Zuordnung zueinander unterschiedlich sein. Im einfachsten Fall besteht ein Pflaster aus senkrechten und waagrechten, vorzugsweise jeweils parallelen Kanalstrukturen der Bereiche (ii), die sich an den Kreuzungspunkten schneiden und entsprechende geometrisch gestaltete Bereiche oder Domänen der Bereiche (i) umschließen, die die Wirkstoffdepots bzw. Behältnisse enthalten. Es wird also ein im Prinzip rechteckiges, quadratisches oder rautenförmiges dreidimensionales Gitter von Kanalstrukturen und Depotstrukturen in der Trägermatrix gebildet. Dabei muss ein Kanal bzw. dessen Rand ebenso wie die angrenzenden Depotstrukturen nicht notwendigerweise gerade sein, sondern er kann auch gewellt, zickzack-förmig oder unregelmäßig ausgerichtet sein.

In einer weiteren Ausführungsform des erfindungsgemäßen Pflasters können die beiden Kavitäten der Bereiche bzw. Domänen (i) und (ii) sich gegenseitig ring-, oval, ellipsen- oder rechteckförmig umschließen, d.h. eine entsprechend geformte Kanalkavität grenzt an eine entsprechend geformte Domäne aus Depotkavitäten, wobei der Abstand zwischen zwei gleichartigen Kavitätsdomänen maximal 25mm, vorzugsweise 5 bis 25mm beträgt. Dabei stehen im einfachsten Fall die einzelnen kanalähnlichen Kavitäten nicht miteinander in Verbindung. Durch Schaffung beispielsweise eines senkrechten und / oder waagrechten Hauptkanals, der die jeweiligen ringartigen Domänen durchschneidet, stehen alle kanalähnlichen Kavitäten des Pflaster dann miteinander in Verbindung.

Alternativ, können in einer weiteren Ausführungsform des erfindungsgemäßen Pflasters die beiden Strukturen der Bereiche (i) und (ii) sich spiral- oder meanderartig umschließen, wobei der Abstand gleichartiger Bereiche wiederum zwischen etwa 5 und 25mm beträgt. In dieser Ausführungsform liegt in der Trägermatrix im Prinzip nur eine Kanalstruktur des Bereiches (ii) und nur eine Depotstruktur des Bereiches (i) vor, wobei auch hier in einer weiteren Variante kreuzende Kanalstrukturen der Bereiche (ii) eingezogen werden können.

Auch in den Ausführungsformen, bei denen sich die kanalähnlichen Kavitäten der Bereiche (ii) nicht kreuzen, besitzen diese Strukturen vorzugsweise blasen-, kalotten- oder kugelförmige Aufweitungen oder Hohlräume, an denen Anschlüsse angebracht sind oder in denen Drainagematerial untergebracht sein kann.

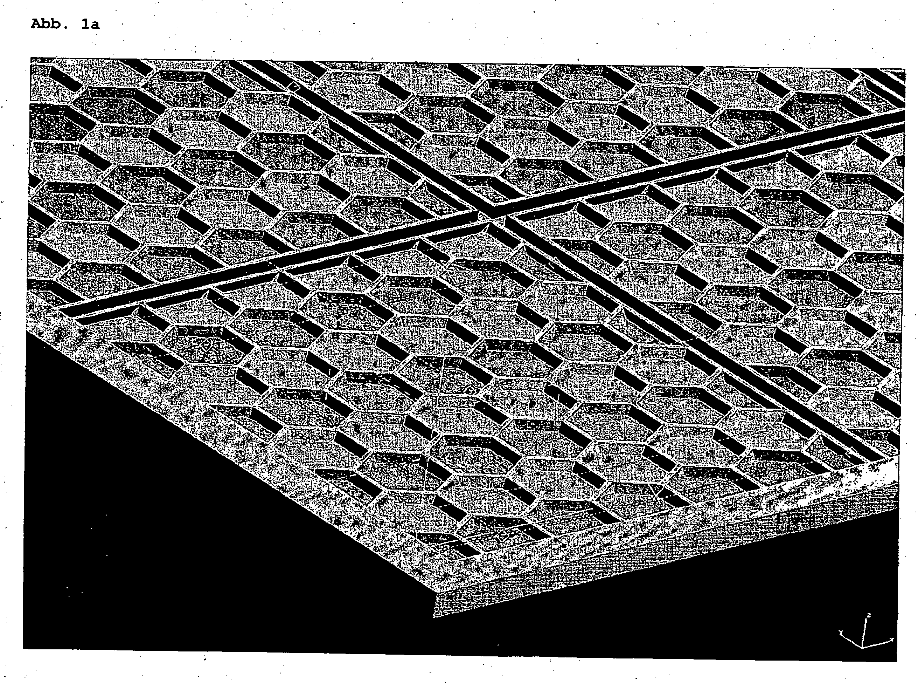

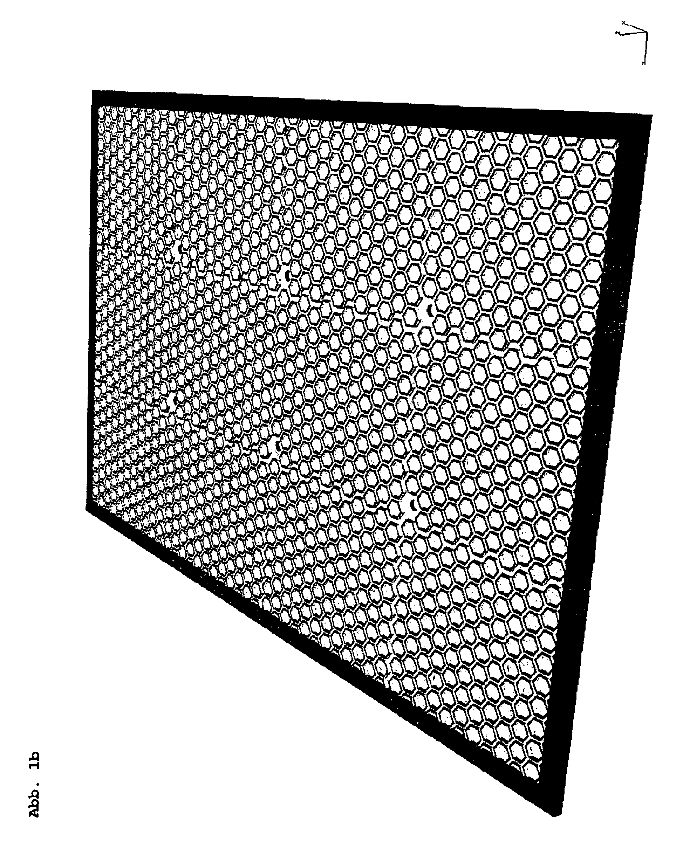

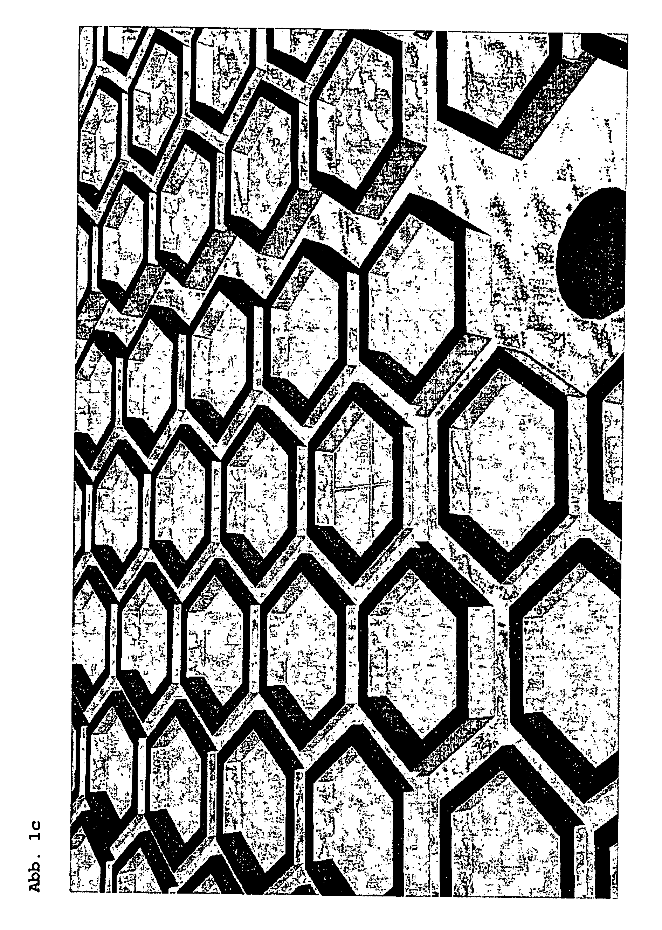

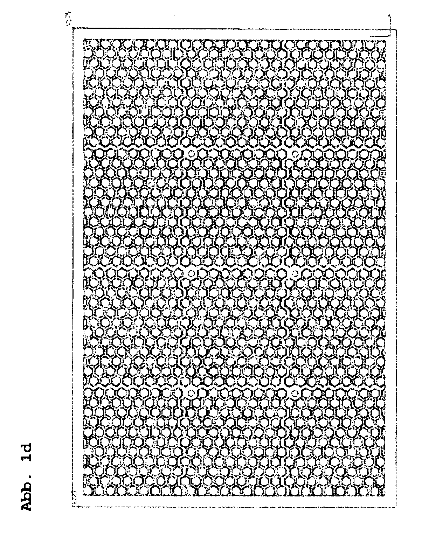

Das Pflaster kann so strukturiert bzw. aufgebaut sein, dass ein einzelner Wirkstoffbehälter vollständig von kanalähnlichen Strukturen umgeben ist. Solche Pflasteranordnungen haben einen auf die Gesamtfläche bezogenen hohen Anteil (20 - 40 %)an Kanalstrukturen und sind insbesondere dann geeignet, wenn viel Wundsekret gleichmäßig entfernt werden muss (Abb. 1c). In vielen Fällen ist es ausreichend, die Depotstrukturen in Form von Clustern zusammenzufassen, wobei kanalähnlichen Strukturen der Bereiche (ii) lediglich zwischen den Clusterstrukturen vorliegen. In diesem Fall ist der Flächenanteil der Kanalstrukturen in der Pflasteranordnung verhältnismäßig klein (2 - 10 %) (Abb. 1a, b, d). Bei dieser Anordnung kann besonders viel Wirkstoff pro Fläche zur Verfügung gestellt werden. Durch VergrößerungNerkleinerung der Kanaldurchmesser bzw. der Dicke/Tiefe der Depots bzw. des Pflasters können so die einzelnen genannten Parameter individuell innerhalb eines bestimmten Spektrums eingestellt werden.

Das erfindungsgemäße Pflaster kann mit einem an sich bekannten selbstklebenden oder selbst haftenden Band, welches an den Rändern des Pflasters angebracht ist, ausgestattet sein, wodurch es möglich wird, die Wunde luftdicht abzuschließen. Ferner wird dadurch auch die Trägermatrix des Pflasters inklusive ihrer kanalähnlichen Strukturen (ii) druckdicht verschlossen, so dass bei Vorhandensein eines Anschlusses für beispielsweise eine Spritze oder einer Absaugvorrichtung Wundsekret unter Erzeugung eines Unterdrucks abgesaugt werden kann. Die Erzeugung eines Unterdruckes bewirkt zudem, dass Wirkstoff aus dem Depot vermehrt freigesetzt und in die nunmehr Wundsekret freie Wunde eingebracht werden kann, um so dort optimal pharmakologisch zu wirken.

Gegenstand ist somit eine entsprechendes Pflaster, das zusätzlich mit einem selbstklebenden Befestigungsmittel ausgestattet ist, welches die Trägermatrix mit dem Arzneimittel / Wirkstoff und somit die Wunde vollständig luft- und druckdicht abschließt.

Das Pflaster kann auch ohne Klebeband oder Klebeverschluss ausgestattet sein und mit anderen Mitteln, beispielsweise einer Bandage auf der Wunde befestigt werden. In diesem Fall kann das Pflaster aufgrund seiner parzellenartigen Mikrodomänenstruktur nicht nur in Bezug auf seine Größe sondern auch in Bezug auf die Wirkstoffmenge, die verabreicht werden soll, zugeschnitten werden. Es ist somit auch eine Dosierung allein über die Pflastergröße möglich, was bislang bei keinem anderen Pflaster des Standes der Technik bekannt ist.

Wird das Pflaster ohne integriertes Klebeband verwendet, so kann Druckdichtigkeit z. B. durch eine gesondertes Klebeband erreicht werden, welches über das Pflaster oder Teilen davon geklebt bzw. befestigt wird.

Muss das erfindungsgemäße Pflaster gänzlich ohne Verwendung eines Klebebandes eingesetzt werden, wie dies beispielsweise bei Anwendung auf der Schleimhaut, z. B. in der Mundhöhle nicht zu vermeiden ist, sollte es zumindest mit Drainagematerial anstelle von einem Anschluss für eine Absaugeinrichtung ausgestattet sein, damit Wundsekret durch die Saugwirkung der Drainage abtransportiert werden kann. So kann das erfindungsgemäße Pflaster auch in Wunden bei Zahnbehandlungen, Zahnextraktionen, Behandlungen des Zahnfachs und Kieferoperationen in Form von zurechtgeschnitten Streifen und "Pads" eingelegt werden. Die für diese aber auch andere Fälle geeignet Drainage kann zum Beispiel in einem Kollagenvlies oder einem Gelatineschwamm oder anderen im Stand der Technik hierfür verwendeten Materialien bestehen. Ein solches Material kann auch verwendet werden um die Wunde mit dem Pflasterelement abzudecken, wobei auch Fibrinkleberpräparate eingesetzt werden können. Gegebenenfalls kann die Schleimhautwunde vernäht werden, um den fibrinolytischen Einfluss des Speichels zu verringern.

Bei besonders trockenen Veränderungen der Haut, wie diese z.B. bei dermatologischen Erkrankungen mit und ohne Erythemen auftreten können, bei Hautrötungen wie z.B. Sonnenbrand oder auch normalen Hautzuständen können die Pflaster auch ohne drainierende Kanäle oder ohne Eröffnung derselben angewendet werden.

In die Depotstrukturen der Bereiche bzw. Domänen (i) kann der Wirkstoff oder die Wirkstoffe in unterschiedlichster Weise eingebracht werden. So kann beispielsweise das Wirkstoff-Protein in fester Form, zu Beispiel in lyophiliserter Form oder ggf. auch als festes Proteinpulver in die einzelnen Kavitäten verpresst werden. Durch die spezielle Geometrie (z.B. wabenförmige Grundfläche) und Größe der Depotkavitäten in der Trägermatrix, was eine relative große Oberfläche im Vergleich zum Volumen zur Folge hat, werden im einfachsten Fall keine zusätzlichen Trägerstoffe benötigt.

Die Depotkammern können den Wirkstoff aber auch in löslicher Form oder als Suspension enthalten. In diesem Fall müssen die Depotdomänen mit einer Schutzfolie versehen sein die vor Auftragen des Pflasters auf die Wunde entfernt wird. Neben einer solchen Schutzfolie, die undurchlässig für jegliche Wirkstoffe und Flüssigkeiten in den Depotkammern ist, und auch vorzugsweise eingesetzt werden kann, wenn der Wirkstoff in fester Form verpresst oder in einem nicht-flüssigen Trägermaterial aufgenommen vorliegt, kann auch erfindungsgemäß eine die Depotkammern unmittelbar abdeckende permeable oder semipermeable Folie vorgesehen sein, welche Moleküle des Protein-Wirkstoffes und / oder ggf. der anderen Wirkstoffe in verzögerter Weise passieren und in die Wunde einwandern lässt, nicht hingegen in umgekehrter Richtung proteolytische Enzyme oder Bakterien aus der Wunde. Solche entsprechenden Folien können auch aus bioabbaubaren Materialien, wie sie unternäher aufgeführt sind, hergestellt sein. Derartige Folien oder Membranen sind im Stand der Technik hinlänglich bekannt.

Gegenstand der Erfindung ist somit ein entsprechendes Pflaster, welches auf der zur Wunde bzw. Haut zugewandten Seite eine Schutzfolie aufweist, die vor Gebrauch entfernt wird.

Gegenstand der Erfindung ist auch ein entsprechendes Pflaster, welches auf der zur Wunde / Haut zugewandten Seite eine Folie oder ein Membran aufweist, die durchlässig für das Arzneimittel ist und ggf. undurchlässig für proteolytische Enzyme oder anderer unerwünschter Stoffe mit größeren Molekulargewichten aus dem Wundsekret ist.

Gegenstand der Erfindung ist auch ein entsprechendes Pflaster, welches eine Folie oder Membran aus einem biologisch abbaubaren Material umfasst.

Der Protein-Wirkstoff kann aber auch in einem Wirkstoff-Reservoir in Form eine porösen Matrix vorliegen. Eine solche poröse Matrix kann ein synthetischer oder natürlicher poröser Träger sein, so zum Beispiel, polymere Substanzen wie Hydroxymethylenacrylate, Polyethylenglykole, Polythylenoxide. Diese Materialien können durch Bestrahlung oder andere bekannte Verfahren quervernetzt werden, um Wasserunlöslichkeit zu gewährleisten.

Besonders geeignet sind auch biokompatible bzw. bioresorbierbare Trägersubstanzen wir Polyzucker, Polyvinylakohole, Polylaktide, Polymilchsäure, Poylvinylpyrrolidon. Poly-L-Lysin oder auch Milchsäure-Glycolsäure-Kopolymer. Die Arzneimittelsubstanzen können aber auch in einem Kollagenschwamm eingebunden sein, welcher in den Depotkammern untergebracht ist.

Die Protein- und anderen Wirkstoffe können aber auch in so genannte Mikrosphären eingebettet, dispergiert oder gelöst enthalten oder auch von diesen umschlossen sein. Mikropartikel gemäß der Erfindung umfassen Liposomen sowie Nanopartikel mit einer Größe zwischen 10nm und 2mm. Geeignete Materialien zur Herstellung von Mikrosphären sind beispielsweise quervernetzte Dextrane, keramische Materalien oder natürlich auch synthetische Polymere, wie Polyurethane, Polymethylmethacrylate, Polyetheylenterephtalate, Polystyrene, Polyolefine, Polyacralamide, Polylaktide, Polyglykolide und andere synthetische Ploymerwerkstoffe, die für biomedizinische Anwendungen geeignet sind. Die entsprechenden Mikrosphären, welche mit dem Wirkstoff beladen sind, werden in die Depotkammern eingebracht, und geben dort den oder die Wirkstoffe in kontrollierter und verzögerter Weise and die Wunde ab.

Neben dem Proteinwirkstoff können, wie bereits mehrfach erwähnt, auch andere Wirkstoffe und auch Zellen, welche die Wundheilung fördern, in den Depotkavitäten der Bereiche (i) zusätzlich vorliegen. Beispielsweise können Proteinaseinhibitoren, wie etwa Aprotinin, oder auch Antibiotika, die hilfreich bei infizierten Wunden sind, dienlich sein. Ebenso können xenogene oder autogene, insbesondere aber autologe Zellpräparationen, die beispielsweise Fibroblasten, Endothelzellen, Makrophagen, Monozyten oder auch Stammzellen einzeln und Gemisch umfassen, die durch das primäre Wirk-Protein initiierte Wundheilung unterstützen.

Als Trägermatrix für das erfindungsgemäße Pflaster sind prinzipiell alle bekannten Pflastermaterialien geeignet, die die Herstellung einer Mikrodomänenstruktur, wie beschrieben, ermöglichen. Besonders geeignet sind Silikonmaterialien oder synthetischer Gummi, Naturkautschuk oder synthetisches Polymermaterial aus den im Stande der Technik für diese und ähnliche Zwecke beschriebenen Stoffe, wie im Prinzip oben für vergleichbare Materialien beschrieben. Die zur Verwendung kommenden Materialien sollten in jedem Fall die Fähigkeit besitzen, entsprechend Verformungen durch Gießen oder Pressen des Materials zu ermöglichen, um die erwähnte Mikrodomänenstruktur erhalten zu können. Außerdem sollten sie verhältnismäßig weich und anschmiegsam sein.

Gegenstand der Erfindung ist somit ein entsprechendes Pflaster, welches eine Trägermatrix für die Kavitäten der Bereiche (i) und (ii) umfasst und diese Trägermatrix vorzugsweise aus einem polymeren Kunststoff, Silikon oder einem Natur-Elastomermaterial angefertigt ist, welches geeignet ist, dass die notwendigen Mikrodomänenstrukturen auf bzw. in der Trägermatrix, beispielsweise durch Schneiden, Gießen, Pressen und ähnlichen Verfahren hergestellt werden können.

Generell eignet sich das erfindungsgemäße Pflaster zum Einsatz für unterschiedlichste pharmakologische Wirkstoffe, vorzugsweise ist aber für Proteine bzw. Polypeptide geeignet, wie einleitend ausführlich diskutiert. Die besonders vorteilhaften Eigenschaften können insbesondere für Proteine und Polypeptide mit einem Molekulargewicht von unter 50 kD, vorzugsweise zwischen 10 und 50 kD erzielt werden, wobei Proteine, die im Prinzip leicht dem Angriff von spaltenden Enzymen oder infizierenden Zellen ausgesetzt sind, sehr gute Ergebnisse im Bezug auf Wirksamkeit und verlängerten Halbwertszeiten liefert. Solche Proteine sind beispielsweise Cytokine und Wachstumsfaktoren wie EGF, TGF beta, GSF, GM-CSF, HGH, CNTF, EPO, TPO, Interferone wie IFN alpha, IFN beta, INF gamma und Interleukine wie z.B. IL-2, IL-7, IL6, IL8, aber auch Blutgerinnungsfaktoren, wie z.B. Thrombin, Hämatin und dergleichen.

Wie bereits beschrieben, zeigt das erfindungsgemäße Pflaster besonders gute Ergebnisse mit dem Wirkstoff Erythropoietin (EPO), seiner Derivate und Fragmente sowie Peptidmimetika und Peptidanaloga mit der biologischen hämatopoietschen und insbesondere nichthämatopoietschen biologischen Aktivität von EPO. Durch ein erfindungsgemäßes mit EPO beladenem Pflaster kann die Wundheilung einer Haut, Schleimhaut oder Fleischwunde entscheidend begünstigt werden.

Dabei hat sich gezeigt, dass EPO in einer wöchentlichen Dosis von über 100, vorzugsweise 100 - 500 IU /kg Körpergewicht ( 1µg rekombinantes humanes EPO entspricht 130 IU) für eine beschleunigte und Wundheilung bei Verletzungen, insbesondere auch im Kiefer und Dentalbereich sorgen kann, wenn es topisch über ein erfindungsgemäßes Pflaster appliziert wird, vorzugsweise in lyophiliserter Form, wobei zusätzlich auch Anteile von lyophiliserten Blutplättchen zugegen sein können. Da ein solches Pflaster zwischen vorzugsweise 48 und 72 Stunden gewechselt werden sollte, ist es notwendig aber ausreichend, dass ein solches Pflaster etwa 25 - 175 IU / kg EPO enthält, damit eine zufrieden stellender Effekt erzielt werden kann. Es können aber auch Dosen pro Pflaster von über 200 IU / kg Körpergewicht und mehr bei zwei- bis dreimaligem Wechseln des Pflaster pro Woche vorgesehen werden. In hoch dosierten Formen eines Pflasters (über 300 IU / kg) kann ein solches Pflaster auch über sieben Tage beibehalten werden. Zu beachten ist, dass erfindungsgemäß letztendlich die enge des Wirkstoffes sowie die Kinetik seiner Freisetzung auch durch die Dicke der Depots gesteuert werden kann.

Für andere Protein-Wirkstoffe können ähnliche Dosen angewandt werden, bzw. sie richten sich nach den Dosen, die auch bei systemischer Gabe üblich sind.

Gegenstand der Erfindung ist somit ferner ein entsprechendes Pflaster, bei dem das in die Kavitäten der Bereiche (i) eingebrachte Arzneimittel ein Polypeptid oder Protein ist, welches vorzugsweise Molekulargewicht von 10 - 50 kD besitzt und gegebenenfalls proteolyseempfindlich ist, bzw. durch in Wunden vorkommenden Enzyme und Bakterien leicht zu spalten ist.

Gegenstand der Erfindung ist insbesondere ein entsprechendes Pflaster, welches einen Wachstumsfaktor oder ein Cytokin, vorzugsweise EPO oder ein peptidisches Molekül mit der biologischen Aktivität von EPO enthält, insbesondere EPO in lyophiliserter Form.

Gegenstand der Erfindung ist letztlich die Verwendung eines Pflasters wie oben und in den Ansprüchen beschrieben, zur Wundabdeckung sowie die Verwendung von EPO und anderer geeigneter Wachstumsfaktoren und Cytokinen zur Herstellung eines Pflasters, wie oben und in den Ansprüchen beschrieben, zur regenerativen Heilung von verletzter Haut, Schleimhaut oder offenen Wunden.

Zusammengefasst, betrifft die Erfindung folgende Gegenstände:
- Ein Pflaster zur Versorgung von verletzter Haut, Schleimhaut oder offenen Wunden mit einem oder mehreren Wirkstoffen oder Arzneimitteln im wesentlichen umfassend eine Trägermatrix, welche den oder die Wirkstoffe oder das oder die Arzneimittel enthält, wobei die Trägermatrix folgendes umfasst:
   (I) Bereiche bzw. Strukturen (i) in Form einer oder mehrerer als Behältnis für das Arzneimittel dienenden Kavitäten, und
   (II) Bereiche bzw. Strukturen (ii) in Form von einer oder mehrerer als kanalähnliche Strukturen dienenden Kavitäten, welche der Aufnahme und Abführung von Wundsekret und / oder Belüftung und / oder der topischen Einbringung von weiteren Wirkstoffen und / oder von Zellen dienen, welche die Heilung der Haut, Schleimhaut oder offenen Wunde fördern, wobei
      (a) mindestens ein Bereich gemäß (i) an mindestens einen Bereich gemäß (ii) grenzt, (b) die Bereiche (i) und (ii) auf der von der Wunde abgewandten Seite verschlossen sind, (c) die Bereiche (i) und (ii) auf der der Wunde zugewandten Seite offen oder für besagte Arzneimittel, Wundsekret, weitere Wirkstoffe/besagte Zellen zumindest passierbar sind, (d) die als Kavitäten ausgebildeten Bereiche (i) und (ii) in der Ebene des Pflasterfläche angeordnet sind, und(e) die Kavitäten der Bereiche (ii) mindestens eine Öffnung oder einen Anschluss für eine Spritze oder Absaug- / Zuführungsvorrichtung aufweisen, der es ermöglicht, entweder unter Erzeugung eines im Pflaster erzeugten Unterdrucks in den Kavitäten angesammeltes Wundsekret abzuführen und /oder gegebenenfalls besagte weitere
      Wirkstoffe zu applizieren.
- Ein entsprechendes oben definiertes Pflaster bei dem im Falle nässender oder Wundsekret produzierender Haut oder Wunden (f) die Kavitäten der Bereiche (ii) zusätzlich Drainagemittel aufweisen, die es ermöglichen, in den Kavitäten angesammeltes Wundsekret abzuführen.
- Ein entsprechendes oben definiertes Pflaster bei dem die Kavitäten der Bereiche (ii) Kanäle sind, die untereinander in Verbindung stehen.
- Ein entsprechendes oben definiertes Pflaster bei dem die als Behältnis für das Arzneimittel dienen Kavitäten der Bereiche (i) wannenförmig sind, wobei die offenen Seite der Wannen zur Wunde hin ausgerichtet ist, und die Wannen von rechteckiger, quadratischer, sechseckiger/wabenförmiger oder runder Grundfläche sind.
- Ein entsprechendes oben definiertes Pflaster bei dem die als Behältnis für das Arzneimittel dienen Kavitäten der Bereiche (i) durch Stege aus dem Material der Trägermatrix voneinander getrennt sind.
- Ein entsprechendes oben definiertes Pflaster bei dem die Stege selbst kanalähnliche Strukturen aufweisen, welche untereinander zumindest teilweise in Verbindung stehen und der Abführung vom Wundsekret, und / oder der Belüftung und / oder der Zuführung von weiteren Wirkstoffen / besagte Zellen dienen.
- Ein entsprechendes oben definiertes Pflaster bei dem die die als Behältnis für das Arzneimittel dienen Kavitäten der Bereiche (i) Substrukturen darstellen, welche in Clustern zusammengefasst sind, die durch Stege aus dem Material der Trägermatrix voneinander getrennt sind.
- Ein entsprechendes oben definiertes Pflaster bei dem die Clusterdomänen eine beliebige, rechteckige, quadratische, sechseckige/wabenförmige oder runde, vorzugsweise eine quadratische oder rechteckige Grundflächenform aufweisen.
- Ein entsprechendes oben definiertes Pflaster bei dem die Form der Clustergrundfläche gleich oder verschieden von der Grundflächenform der Substrukturen der Bereiche (i) ist.
- Ein entsprechendes oben definiertes Pflaster bei dem mindestens eine Clusterdomäne des Bereiches (i) an mindestens eine Kavität des Bereiches (ii) grenzt.
- Ein entsprechendes oben definiertes Pflaster bei dem zwei Clusterdomänen der Bereiche (i) durch eine Kavität des Bereiches (ii) von einander getrennt sind.
- Ein entsprechendes oben definiertes Pflaster bei dem sich eine Kavität der Bereiche (ii) mit mindestens einer anderen Kavität der Bereiche (ii) schneidet.
- Ein entsprechendes oben definiertes Pflaster bei dem die Kavitäten der Bereiche (ii) ein rechteckiges, quadratisches oder rautenförmiges Netz oder Gitter in der Trägermatrix bilden, wobei sie Strukturen der Bereiche (i) umschließen.
- Ein entsprechendes oben definiertes bei dem die Strukturen der Bereiche (i) Clusterdomänen wie oben und in den Ansprüchen näher beschrieben sind.
- Ein entsprechendes oben definiertes Pflaster bei dem die Strukturen der Bereiche (i) und (ii) die Form einer rechteckigen oder runden Spirale oder eines rechteckigen oder runden Ringes angeordnet sind.
- Ein entsprechendes oben definiertes Pflaster bei dem die Trägermatrix eine oder mehrere besagte spiral- oder ringförmigen Anordnungen aufweist, so dass ihre Fläche im wesentlichen durch diese Anordnung(en) ausgefüllt ist.
- Ein entsprechendes oben definiertes Pflaster bei dem die Kavitäten der Bereiche (i), welche das Arzneimittel enthalten, eine Dicke besitzen, welcher der zu erzielenden Depotwirkung des Arzneimittels angepasst ist.
- Ein entsprechendes oben definiertes Pflaster bei dem die Dicke bzw. Tiefe der arzneimittelhaltigen Kavitäten der Bereiche (i) 0.3 bis 3.0 mm beträgt.
- Ein entsprechendes oben definiertes Pflaster bei dem eine einzelne Kavität der Bereiche (i) einen mittleren lichten Durchmesser von 0.5 bis 5.0 mm aufweist.
- Ein entsprechendes oben definiertes Pflaster bei dem die einzelnen Kavitäten der Bereiche (i) in Clustern gemäß der Ansprüche 5 bis 8 zusammengefasst sind, welche einen mittleren lichten Durchmesser von 5.0 bis 25.0 mm besitzen.
- Ein entsprechendes oben definiertes Pflaster bei dem die einzelnen das Arzneimittel enthaltenden Kavitäten der Bereiche (i) in Clustern wie beschrieben, zusammengefasst sind, wobei jede Clusterdomäne 20 bis 200 wannenähnliche Einzelstrukturen der Bereiche (i) aufweist.
- Ein entsprechendes oben definiertes Pflaster bei dem die kanalähnlichen Kavitäten der Bereiche (ii) einen mittleren lichten Durchmesser von 0.3 bis 3.0 mm aufweisen.
- Ein entsprechendes oben definiertes Pflaster bei dem der Abstand zwischen zwei kanalähnlichen Kavitäten der Bereiche (ii) und der Abstand zwischen zwei Clusterdomänen der Bereiche (i) jeweils 5 bis 25 mm beträgt.
- Ein entsprechendes oben definiertes Pflaster bei dem bei sich schneidenden kanalähnlichen Kavitäten der Bereiche (ii) der Anschluss für eine Spritze oder Absaug-/ Zuführungs-vorrichtung im Kreuzungsbereich zweier Kanalstrukturen (i) angebracht ist.
- Ein entsprechendes oben definiertes Pflaster bei dem bei sich schneidenden kanalähnlichen Strukturen der Bereiche (ii) der Kreuzungsbereich in Form eine Blase, Kalotte oder Kugel vergrößert ist.
- Ein entsprechendes oben definiertes Pflaster, welches auf der zur Wunde / Haut zugewandten Seite eine Schutzfolie aufweist, die vor Gebrauch entfernt wird.
- Ein entsprechendes oben definiertes Pflaster, welches auf der zur Wunde / Haut zugewandten Seite eine Folie oder ein Membran aufweist, welche durchlässig für das Arzneimittel ist.
- Ein entsprechendes oben definiertes Pflaster bei dem die Folie oder Membran im wesentlichen undurchlässig für proteolytische Enzyme aus dem Wundsekret ist.
- Ein entsprechendes Pflaster bei dem die Folie oder Membran aus einem biologisch abbaubaren Material angefertigt ist.
- Ein entsprechendes oben definiertes Pflaster bei dem die Trägermatrix inklusive ihrer Strukturen der Bereiche (i) und (ii) aus einem polymeren Kunststoff, Silikon oder einem Natur-Elastomer angefertigt ist.
- Ein entsprechendes oben definiertes Pflaster bei dem die Trägermatrix inklusive ihrer Strukturen der Bereiche (i) und (ii) aus einem bioabbaubaren Material angefertigt ist.
- Ein entsprechendes Pflaster, welches weiterhin ein selbstklebendes Befestigungsmittel umfasst, das die Trägermatrix mit dem Arzneimittel / Wirkstoff und somit die Wunde vollständig luft- und druckdicht abschließt.
- Ein entsprechendes oben definiertes Pflaster bei dem das in die Kavitäten der Bereiche (i) eingebrachte Arzneimittel ein Polypeptid oder Protein ist.
- Ein entsprechendes oben definiertes Pflaster bei dem das in die Strukturen (i) eingebrachte Arzneimittel ein Polypeptid oder Protein mit einem Molekulargewicht von 10 - 50 kD ist.
- Ein entsprechendes Pflaster bei dem das Polypeptid oder das Protein proteolyseempfindlich ist.
- Ein entsprechendes oben definiertes Pflaster bei dem das Polypeptid oder Protein ein Wachstumsfaktor oder Cytokin ist.
- Ein entsprechendes oben definiertes Pflaster bei dem das Polypeptid oder Protein Erythropoietin (EPO) oder ein biologisch aktives Derivat oder Fragment davon, vorzugsweise in lyophilisierter Form, ist.
- Eine Verwendung eines Wachstumsfaktors oder Cytokins, welcher/welches für die gezielte Geweberegenration zu fördern vermag, zur Herstellung eines oben oder unten beschriebenen Pflasters für die Wundabdeckung bei Haut- und Schleimhautverletzungen.
- Eine Verwendung von EPO zur Herstellung eines beschriebenen Pflasters zur regenerativen Heilung von verletzter Haut, Schleimhaut oder offenen Wunden.

### KURZE BESCHREIBUNG DER ABBILDUNGEN:

Abbildung 1 zeigt die Struktur eine Ausführungsform des erfindungsgemäßen Pflasters in dreidimensionaler Sicht. Die Depots für den Wirkstoff haben wabenförmige Struktur und sind zu Clustern von etwa 50 Einzeldepots zusammengefasst, die durch gitterartige Kanäle von einander getrennt sind.
Abbildung 1b zeigt eine ähnliche Ausführungsform wie Abb. 1. Die Kanäle sind in eckiger Linienführung entsprechend der Wabenstruktur der Depots ausgebildet. An den Kreuzungspunkten der Kanäle sind Öffnungen zum anschließen einer Spritze oder einer Absaugvorrichtung oder einer Zuführungsvorrichtung vorgesehen.
Abbildung 1c zeigt den Ausschnitt eines erfindungsgemäßen Pflasters mit Waben als Depot für den Wirkstoff, jedoch ist jedes einzelne Depot komplett von Kanalkavitäten umgeben. Am unteren rechten Rand ist eine erweiterte Kreuzungszone mit einer Öffnung zum Abführen oder Zuführen abgebildet.
Abbildung 1d zeigt ein erfindungsgemäßes Pflaster analog Abb. 1a in Originalgröße.

### ANWENDUNGSBEISPIELE DES ERFINDUNGSGEMÄSSEN PFLASTERS:

(1) Für eine tiefe Schürfwunde von einer Größe von etwa vier x fünf Zentimetern wird ein Pflaster aus einer größeren Pflastervorlage von etwa 8 x 12 Zentimetern herausgeschnitten. Das Pflaster weist eine Domänenstruktur gemäß der Abb. 1d auf mit den folgenden Maßen: wabenförmige Einzeldepots mit einer Tiefe von 1 mm und einem lichten Durchmesser von 2.5mm. Jeweils 80 solcher Einzeldepots sind zu Clustern zusammengefasst, welche wiederum von Kanälen von 1mm Tiefe und 1.5mm Breite voneinander getrennt sind.

Die Kanäle haben Gitterstruktur und besitzen an einigen Kreuzungspunkten eine verschließbare Öffnung, an dem eine Absaugvorrichtung angeschlossen werden kann. Die Einzeldepots enthalten insgesamt 13.000 IU EPO (entspricht etwa 100 µg). Das EPO wurde zuvor in löslicher flüssiger Form in die aus Weichsilikon gefertigte Trägerstruktur des

Pflasters eingebracht, beziehungsweise gleichmäßig auf die Clusterstrukturen verteilt. Die Lyophilisierung des Wirkstoffes erfolgte auf dem Pflaster, bzw. in den Depotbehältern. Das so gefertigte Pflaster wurde auf der offenen Seite mit eine Schutzfolie versehen, welche vor Aufbringen auf die Wunde entfernt wurde. Das Pflaster wurde auf die frische aber nicht mehr blutende Wunde aufgebracht, und mit einem Klebeband auf der gesunden Haut befestigt und luftdicht abgeschlossen. Anfangs alle drei, später alle sechs Stunden wurde mittels einer kleinen Spritze, die auf eine der Anschlüsse auf dem Pflaster gesteckt wurde, Wundsekret abgesaugt. Nach 48 Stunden wurde das Pflaster gewechselt. Absaugen des Wundsekretes erfolgte danach nur noch 1 - 2 mal innerhalb von 24 Stunden. Ein erneuter Wechsel des Pflasters wurde nach weiteren drei Tagen vorgenommen, wobei nur noch einmal abgesaugt wurde. Jeweils beim Wechsel des Pflasters wurde der Heilungszustand überprüft und charakterisiert.

Im Vergleich hierzu, wurde auf eine vergleichbare Wunde ein Gelformulierung, welche EPO in der gleichen Dosis wie oben angegeben enthielt, auf die Wunde aufgetragen. Nach den gleichen Zeitabschnitten wie bei dem Pflaster wurde die Gelauftragung erneuert und der Heilungsfortschritt beobachtet. Die Wunde mit dem Pflaster heilte in jeder Phase schneller und war schließlich schon nach 60 - 80% der Zeit verheilt im Vergleich zu der entsprechenden Gelformulierung.

(2) Bei einem Sonnenbrand mit starker Rötung der Haut und beginnenden Abschälungen wird das Pflaster mit einem in den Kavitäten enthaltenen Hydrogel das EPO in einer Konzentration von 100 IU/ Kg und Woche enthält unter Bedeckung der geschädigten Areale großflächig aufgelegt. Die Weichheit des verwendeten Silikonmaterials ergibt eine anschmiegenden und sofort schmerzstillenden Effekt da der Feuchtigkeitsverlust der Haut vermieden wird und vor Ort durch den Rückbehalt in Kombination mit einem z.B. verwendeten Methylcellulosegel auf der Haut angenehm kühlt. Zur Stimulierung der in den Haarfollikeln im dermalen Bereich noch vitalen Stammzellen der Haut kann nun EPO direkt auf diese Zellen einwirken und die Regeneration der Haut beschleunigen ohne den Umweg über die systemische Applikation gehen zu müssen. Hierdurch werden die polycythämischen Wirkungen vermieden. EPO wird in der Haut zugleich abgebaut. Die Heilung des Sonnenbrandes wird beschleunigt so dass nach 2-3 Tagen schon ein optimaler Verlauf erkennbar ist, während bei der Anwendung des gleichen Hydrogels mit de gleichen Konzentration EPO ohne Pflaster der gleiche Effekt erst nach 4 - 5 Tagen zu beobachten ist.

(3) Bei schlecht heilenden Wunden wie z.B. Ulzera bei Diabetes kann das Pflaster nach einer Säuberung der Wunde dem so genannten Debridement direkt auf den Wundbereich mit einer Überlappung zu noch intakten Bereich aufgebracht werden. Diese schlecht heilenden Wunden besitzen jedoch noch Stammzellen wie CD90+ Zellen, die neben den CD31+ Zellen in den Wundarealen vorhanden sind. Die direkte Stimulierung dieser Zellen ermöglicht eine rasche Einstellung der therapeutische Wirkstoffkonzentration ohne über den Umweg einer systemischen Applikation gehen zu müssen. Dies hat den Vorteil dass gerade bei diesen chronischen Wunden Akkumulationsphänomene und Risiken der systemischen Applikation vermieden werden. Die so genannten subpolycythämischen Dosierungen von unter 90 U/KGW verlieren hier an Bedeutung, da einerseits nicht nur die Endothelvorläuferzellen stimuliert werden sollen, sondern vor allem die ortsständigen gewebespezifischen Vorläuferzellen für die Haut mit den Anteilen, Dermis, Hautanhangsgebilden sowie Epidermis zur einer narbenfreien Heilung angeregt werden sollen. Die Dosierung kann somit als Units / pro Fläche berechnet werden. Es hat sich gezeigt, dass insbesondere die gewebespezifischen Stammzellen eine Dosierung erheblich über der so genannten subpolycythämischen Dosierung benötigen um optimal und klinisch relevant stimuliert zu werden. Der Anteil der Gefäße an dem Volumen der Haut liegt nur bei einigen wenigen Prozent. Mehr als 90-95% sind jedoch spezifische Gewebestrukturen. Durch das Zusammenspiel einer starken regionalen Konzentration können alle ortständigen Stammzellen rasch und deutlich nicht nur angesprochen werden sondern auch reagieren. Die systemische Applikation würde ja alle Zellen auf dem Weg zum Wirkort schon ansprechen müssen und daher nicht nur Endothelzellen sondern jegliche Zellen mit diesem Rezeptor adressieren. Es wurde gefunden, dass die Zellen in der Haut den für die Gewebeprotektion verantwortlichen Rezeptor (betaCR) tragen, und dies keine Endothelzellen sind. Der Vorteil der topischen Applikation mit dem Pflaster besteht nun zusätzlich noch darin, dass neben der kontrollierten Entfernbarkeit von Wundsekret davon unbeeinflusst der Wirkstoff direkt in den Wundbereich eingebracht werden kann. Hierzu kann dieser über Gel oder Salben, Mizellen und Lipogelapplikationen z.B. eingebracht werden. In einer vereinfachten Varianten können die Kavitäten die Wirkstoffe aber auch in trockener z.B. lyophilisierter Form enthalten. Dies ermöglicht dann eine besonders innige Verbindung mit Plasma - und Blutbestandteilen auf der frisch gesäuberten Wunde. Die dadurch ausgelöste Polymerisation schafft ein besonders die Wundheilung förderliches Mikromilieu, welches frei von störenden Faktoren ist und somit optimierte Bedingungen der regionalen Stammzelldifferenzierung liefert, die letztendlich ein strukturelles Regenerat hoher Qualität narbenfrei ermöglicht. Da die Gefäßstimulation nur einen initiierenden Anteil an diesem Geschehen hat, kann die topische Applikation mit der verbesserten Integration der gewebespezifischen Stammzellen zu einem fast normalen Regenerat führen. Chronische Wunden, die zuvor oft über Jahre hinweg nicht heilten, heilen nun in 1-2 Wochen Pflasterapplikation ab. Zusätzlich kann die Wunde dadurch auch mit einem einzigen System trocken und steril gehalten werden. Das in der Anfangsphase noch auftretende Wundsekret kann durch den Patienten durch den Anschluss einer 50 ml Spritze an einem der Ports, bzw. Anschlüsse sauber gehalten werden. Zusätzlich können Aprotinin und / oder Antibiotika aus den Depots wie oben erwähnt einwirken.

Der Arzt oder Patient kann in einfacher Form die Wunden auch spülen ohne Gefahr laufen zu müssen, gleich allen Wirkstoff zu entfernen.

*(4) Verbrennungswunden 2. Grades oder Donorstellen der Haut nach Spalthautentnahme:* Bei Wunden bis tief 2. Grades gibt es noch Stammzellen in der Haut, die auf die direkte Applikation von EPO zu einer narbenfreien oder narbenarmen Heilung der Haut zurückgeführt werden können. Nach Entnahmen von Spalthaut oder auch nach der so genannten dermalen Abrasion bei kosmetischen Eingriffen zur Narbenkorrektur im Gesicht kann das anschmiegsame Pflaster auf die anfangs noch leicht nässende Wunde direkt geben werden. Das Silikonmaterial des Pflasters ermöglicht eine Feuchtigkeitsregulierung bei gleichzeitiger schonender Absaugmöglichkeit von Wundsekreten. Parallel kann lyophilisiertes EPO sich direkt mit ortständigen Plasma verbinden und somit eine optimales, die Stammzellen aktivierendes Wundmilieu schaffen. Die protrahierte Freisetzung ermöglicht bei diesen Wunden eine Hinauszögerung des Verbandwechsels. Eine Spalthaut-entnahme kann somit in 3-4Tagen statt nach 9-10 Tagen abheilen. Bei der Verwendung von transparenten Materialien für das Pflaster z.B. Silikon, PDMS, oder auch weicht Teflonverbindungen kann der Heilungsverlauf sogar direkt ohne Interferierung mit dem sterilen Milieu unterhalb des Pflasters beobachtet werden. Der Arzt kann dadurch rasch und nicht-invasiv auf regionale Verhältnisse seine Diagnose abstimmen und Patienten schonend sowie Ressourcen schonend handeln.

*(5) Stammzelltherapie insbesondere bei tiefen Verbrennungen dritten Grades:*
Das Pflaster kann in einer vorteilhaften Anwendungsform auch dazu benutzt werden Stammzellen aus dem Knochenmark, Fettgewebe, Blut oder anderen Hautbereichen direkt und aus therapeutischen Gründen in den erkrankten Bereich einzubringen. Dies ist dann besonders indiziert, wenn durch den Schwergrad der Verletzung keine oder kaum noch ortständige Stammzellen vorhanden sind. Dies ist z.B. bei Verbrennungen dritten Grades der Fall, da dort die dermalen Anteile vollständig zerstört sind. Trotz des Vorhandenseins von Gefäßvorläuferzellen aus den darunter liegenden Muskelanteilen kann die Haut trotzdem nicht regenerieren. Dies zeigt auch die Bedeutung der topischen Stimulierbarkeit der Hautvorläuferzellen durch EPO neben den allgemeinen und nicht immer wünschenswerten systemischen Stimulierungen von Endothelvorläuferzellen bei der systemischen Gabe von EPO mit oder ohne subpolycythämischen Dosierungen. Dieser Effekt alleine reicht keinesfalls aus, eine derartige Wunde zum Heilen zu bringen.
In einer vorteilhaften Anwendung der Erfindung können Stammzellen über die Ports exogen zugeführt werden. Das Pflaster wird hier wie ein aufgeklebter Bioreaktor verwendet. Das EPO aus den Mikrodomänen wird langsam freigesetzt und verbindet sich mit den aus dem Knochenmark eingeführten Stammzellen. Diese werden über eine Nadelaspiration (10-50 ml) gewonnen und durch Zentrifugation auf 1-2 ml Volumen reduziert. Dieses wird langsam über die Ports in tropfenförmiger Art über die Wundfläche verteilt gegeben. Hierdurch wird eine hohe Konzentration von Stammzellen dezentral auch über größere Wundflächen eingebracht. Innerhalb von 1-2 Stunden adhärieren diese Zellen und können danach durch die EPO Exposition gezielt in den Regenerationsprozess geführt werden. Das regionale Verletzungsmilieu fördert dieses Wachstum noch zusätzlich. Über die Sauerstoffverfügbarkeit eines dann vorteilhafterweise besonderes dünnen Silikonpflasters /ca. 50-100 µ Materialstärke können die Zellen in der Anfangsphase oxygeniert werden. Hierdurch kommt es trotz sterilen Wundverschluss zu einer polarisierten Verfügbarkeit von atmosphärischem Sauerstoff wie bei normaler Haut und damit günstigen Differenzierungsbedingungen der Vorläuferzellen in Epidermis und Dermis. Schwerste Verletzungen die sonst nur mit Spalthauttransplantation oder Fremdhauttransplantationen behandelt werden können, heilen nun in 1-2 Wochen ab. Alternativ zu Zellen aus dem Knochenmark können auch Stammzellen aus Haarwurzel mit und ohne vorherige in vitro Expansion in die Ports injiziert werden. Ebenso können kombiniert zum Wiederaufbau des subdermalen Fettgewebes, Stammzellen aus dem Fettgewebe und Fettzellen aus anderen Bereichen des Körpers injiziert werden. Die Mikrostrukturierung des Pflasters ist hier dann von sekundärer Bedeutung wenn der Wirkstoff mit den Stammzellen oder separat injiziert wird oder Bestandteil eines Trägermaterials für die Stammzellen ist (z.B. Hyaluronsäure, Hydrogele, Fibrin, Plasma).

*(6) Schmerztherapie*
Bei chronischen Patienten, die sich häufig auch zu Hause eine variable Dosierung entsprechend den Schmerzerfordernissen und wegen Nebenwirkungen überlegen müssen spielt die Kontrollierbarkeit von Dosierungen eine große Rolle. Konventionelle Pflaster enthalten eine definierte Wirkstoffkonzentration und sind nicht teilbar ohne Depots zu beschädigen und ohne die Gesamtwirkung und Kontrolle damit zu gefährden. Bei Tabletten sind Teilungen jedoch üblich. Kinder bekommen z..B eine halbe Tablette usw. Der Vorteil des dargestellten Pflasterprinzips ist, dass Konzentrationen über Fläche nicht nur der Wunde sondern auch über die Fläche des Pflasters hochpräzis und mit großen Sicherheitsbereichten eingestellt werden können. Ein Pflaster mit einer Fläche von z.B. 0.5 qm Fläche kann ohne weiteres durch den Patienten in 4 gleiche Teile zerteilt werden. Hierzu können in vorteilhafter Form die Kanäle wir Sollbruchstellen aufgebaut werden die eine einfache manuelle Zerteilung wie bei einer Tablette ermöglicht.
Schwierig und variable zu dosierende Wirkstoffe wie z.B. Antibiotika, Schlafmittel, Nikotin, Morphine, ASS und die gesamte Gruppe der Analgetika können dadurch einfach kontrollierbar für den Patienten dosiert werden. Zur Verbesserung der Hautfreundlichkeit und
der Resorption können diese Stoffe in Mizellen in die Depots eingebracht werden. Durch Erwärmung im Körperkontakt können sich die Trägermaterialien verflüssigen oder erweichen und dadurch kontrolliert bei Bedarf und flächengerecht dosiert sowohl regional wirkende Substanzen wie z.B. Antibiotika bei Hautinfektionen als auch Stoffe mit systemischen Wirkungen freisetzen. Im Unterschied zur Wundsituation spielt hier der Proteaseabbau keinen wesentliche Rolle, so dass systemische Wirkungen nicht behindert werden.

## Patentansprüche

1. Pflaster zur Versorgung von verletzter Haut, Schleimhaut oder offenen Wunden mit einem oder mehreren Wirkstoffen oder Arzneimitteln im wesentlichen umfassend eine Trägermatrix, welche den oder die Wirkstoffe oder das oder die Arzneimittel enthält, **dadurch gekennzeichnet, dass** die Trägermatrix umfasst:
(I) Bereiche bzw. Strukturen (i) in Form einer oder mehrerer als Behältnis für das Arzneimittel dienenden Kavitäten, und
(II) Bereiche bzw. Strukturen (ii) in Form von einer oder mehrerer als kanalähnliche Strukturen dienenden Kavitäten, welche der Aufnahme und Abführung von Wundsekret und / oder Belüftung und / oder der topischen Einbringung von weiteren Wirkstoffen und / oder von Zellen dienen, welche die Heilung der Haut, Schleimhaut oder offenen Wunde fördern, wobei
(a) mindestens ein Bereich gemäß (i) an mindestens einen Bereich gemäß (ii) grenzt,
(b) die Bereiche (i) und (ii) auf der von der Wunde abgewandten Seite verschlossen sind,
(c) die Bereiche (i) und (ii) auf der der Wunde zugewandten Seite offen oder für besagte Arzneimittel, Wundsekret, weitere Wirkstoffe/besagte Zellen zumindest passierbar sind,
(d) die als Kavitäten ausgebildeten Bereiche (i) und (ii) in der Ebene des Pflasterfläche angeordnet sind, und
(e) die Kavitäten der Bereiche (ii) mindestens eine Öffnung oder einen Anschluss für eine Spritze oder Absaug- / Zuführungsvorrichtung aufweisen, der es ermöglicht, entweder unter Erzeugung eines im Pflaster erzeugten Unterdrucks in den Kavitäten angesammeltes Wundsekret abzuführen und /oder gegebenenfalls besagte weitere Wirkstoffe zu applizieren.

2. Pflaster nach Anspruch 1, **dadurch gekennzeichnet, dass** die Kavitäten der Bereiche (ii) Kanäle sind, die untereinander in Verbindung stehen.

3. Pflaster nach Anspruch 1 oder 2, **dadurch gekennzeichnet, dass** die als Behältnis für das Arzneimittel dienen Kavitäten der Bereiche (i) wannenförmig sind, wobei die offenen Seite der Wannen zur Wunde hin ausgerichtet ist, und die Wannen von rechteckiger, quadratischer, sechseckiger/wabenförmiger oder runder Grundfläche sind.

4. Pflaster nach einem der Ansprüche 1 bis 3, **dadurch gekennzeichnet dass** die als Behältnis für das Arzneimittel dienen Kavitäten der Bereiche (i) durch kompakte oder kanalähnliche Stege aus dem Material der Trägermatrix voneinander getrennt sind.

5. Pflaster nach einem der Ansprüche 1 bis 4, **dadurch gekennzeichnet, dass** die die als Behältnis für das Arzneimittel dienen Kavitäten der Bereiche (i) Substrukturen darstellen, welche in Clustern zusammengefasst sind, die durch kompakte oder kanalähnliche Stege aus dem Material der Trägermatrix voneinander getrennt sind.

6. Pflaster nach Anspruch 5, **dadurch gekennzeichnet, dass** die Form der Clustergrundfläche gleich oder verschieden von der Grundflächenform der Substrukturen der Bereiche (i) ist.

7. Pflaster nach Anspruch 5 oder 6, **dadurch gekennzeichnet, dass** eine Clusterdomäne des Bereiches (i) an mindestens eine Kavität des Bereiches (ii) grenzt.

8. Pflaster nach einem der Ansprüche 1 bis 7, **dadurch gekennzeichnet, dass** sich eine Kavität der Bereiche (ii) mit mindestens einer anderen Kavität der Bereiche (ii) schneidet.

9. Pflaster nach Anspruch 8, **dadurch gekennzeichnet, dass** die Kavitäten der Bereiche (ii) ein rechteckiges, quadratisches oder rautenförmiges Netz oder Gitter in der Trägermatrix bilden, wobei sie Strukturen der Bereiche (i) umschließen.

10. Pflaster nach Anspruch 9, **dadurch gekennzeichnet, dass** die Strukturen der Bereiche (i) Clusterdomänen gemäß der Ansprüche 5 bis 7 sind.

11. Pflaster nach einem der Ansprühe 1 bis 10, **dadurch gekennzeichnet, dass** die Kavitäten der Bereiche (i), welche das Arzneimittel enthalten, eine Dicke besitzen, welcher der zu erzielenden Depotwirkung des Arzneimittels angepasst ist.

12. Pflaster nach einem der Ansprühe 1 bis 11, **dadurch gekennzeichnet, dass** eine einzelne Kavität der Bereiche (i) einen mittleren lichten Durchmesser von 0.5 bis 5.0 mm aufweist.

13. Pflaster nach einem der Ansprüche 1 bis 12, **dadurch gekennzeichnet, dass** die einzelnen Kavitäten der Bereiche (i) in Clustern gemäß der Ansprüche 5 bis 7 zusammengefasst sind, welche einen mittleren lichten Durchmesser von 5.0 bis 25.0 mm besitzen.

14. Pflaster nach einem der Ansprüche 1 bis 13, **dadurch gekennzeichnet, dass** die einzelnen das Arzneimittel enthaltenden Kavitäten der Bereiche (i) in Clustern gemäß der Ansprüche 5 bis 7 zusammengefasst sind, wobei jede Clusterdomäne 20 bis 200 wannenähnliche Einzelstrukturen der Bereiche (i) aufweist.

15. Pflaster nach einem der Ansprüche 1 bis 14, **dadurch gekennzeichnet, dass** die Kavitäten der Bereiche (ii) kanalähnlich sind und einen mittleren lichten Durchmesser von 0.3 bis 3.0 mm aufweisen.

16. Pflaster nach einem der Ansprüche 1 bis 15, **dadurch gekennzeichnet, dass** der Abstand zwischen zwei kanalähnlichen Kavitäten der Bereiche (ii) und der Abstand zwischen zwei Clusterdomänen der Bereiche (i) jeweils 5 bis 25 mm beträgt.

17. Pflaster nach einem der Ansprüche 1 bis 16, **dadurch gekennzeichnet, dass** bei sich schneidenden kanalähnlichen Kavitäten der Bereiche (ii) der Anschluss für eine Spritze oder Absaug-/Zuführungsvorrichtung im Kreuzungsbereich zweier Kanalstrukturen (i) angebracht ist.

18. Pflaster nach einem der Ansprüche 1 bis 17, **dadurch gekennzeichnet, dass** bei sich schneidenden kanalähnlichen Strukturen der Bereiche (ii) der Kreuzungsbereich in Form eine Blase, Kalotte oder Kugel vergrößert ist.

19. Pflaster nach einem der Ansprüche 1 bis 18, **dadurch gekennzeichnet, dass** es auf der zur Wunde / Haut zugewandten Seite eine Schutzfolie aufweist, die vor Gebrauch entfernt wird.

20. Pflaster nach einem der Ansprüche 1 bis 19, **dadurch gekennzeichnet, dass** es auf der zur Wunde / Haut zugewandten Seite eine Folie oder ein Membran aufweist, welche durchlässig für das Arzneimittel und im wesentlichen undurchlässig für proteolytische Enzyme aus dem Wundsekret ist.

21. Pflaster nach einem der Ansprüche 1 bis 20, **dadurch gekennzeichnet, dass** die Trägermatrix inklusive ihrer Strukturen der Bereiche (i) und (ii) aus einem polymeren Kunststoff, Silikon, einem Natur-Elastomer oder aus einem bioabbaubaren Material angefertigt ist.

22. Pflaster nach einem der Ansprüche 1 bis 21, **dadurch gekennzeichnet, dass** das in die Kavitäten der Bereiche (i) eingebrachte Arzneimittel ein Wachstumsfaktor ist.

23. Pflaster nach Anspruch 22, **dadurch gekennzeichnet, dass** der Wachstumsfaktor Erythropoietin (EPO) in flüssiger oder lyophiliserter Form oder ein biologisch aktives Derivat oder Fragment davon ist.

24. Verwendung von EPO zur Herstellung eines Pflasters gemäß der der Ansprüche 1 bis 23 zur regenerativen Heilung von verletzter Haut, Schleimhaut oder offenen Wunden.

## Claims

1. Plaster for the supply of damaged skin, mucous membrane or open wounds with one or more active compounds or medicaments, essentially comprising a support matrix which contains the active compound(s) or the medicament(s), **characterised in that** the support matrix includes:
(I) regions or structures (i) in the form of one or more cavities serving as container for the medicament, and
(II) regions or structures (ii) in the form of one or more cavities serving as channel-like structures which serve for the accommodation and drainage of wound secretion and / or airing and / or topical introduction of further active compounds and / or of cells which promote healing of the skin, mucous membrane or open wound, where
(a) at least one region in accordance with (i) is adjacent to at least one region in accordance with (ii),
(b) regions (i) and (ii) are closed on the side facing away from the wound,
(c) regions (i) and (ii) are open on the side facing the wound or are at least permeable for said medicaments, wound secretion, further active compounds / said cells,
(d) regions (i) and (ii) formed as cavities are arranged in the plane of the plaster surface, and
(e) the cavities of regions (ii) have at least one opening or connection for a syringe or suction / supply device which enables either wound secretion which has collected in the cavities to be drained with generation of a reduced pressure generated in the plaster and / or any said further active compounds to be applied.

2. Plaster according to Claim 1, **characterised in that** the cavities of regions (ii) are channels which are connected to one another.

3. Plaster according to Claim 1 or 2, **characterised in that** the cavities of regions (i) serving as container for the medicament are in the form of troughs, where the open side of the troughs faces the wound, and the troughs have a rectangular, square, hexagonal / honeycomb-shaped or round base area.

4. Plaster according to one of Claims 1 to 3, **characterised in that** the cavities of regions (i) serving as container for the medicament are separated from one another by compact or channel-like webs comprising the material of the support matrix.

5. Plaster according to one of Claims 1 to 4, **characterised in that** the cavities of regions (i) serving as container for the medicament represent sub-structures which are combined in clusters which are separated from one another by compact or channel-like webs comprising the material of the support matrix.

6. Plaster according to Claim 5, **characterised in that** the shape of the cluster base area is the same as or different from the shape of the base area of the sub-structures of regions (i).

7. Plaster according to Claim 5 or 6, **characterised in that** a cluster domain of region (i) is adjacent to at least one cavity of region (ii).

8. Plaster according to one of Claims 1 to 7, **characterised in that** a cavity of regions (ii) intersects with at least one other cavity of regions (ii).

9. Plaster according to Claim 8, **characterised in that** the cavities of regions (ii) form a rectangular, square or rhomboidal network or grid in the support matrix, surrounding structures of regions (i).

10. Plaster according to Claim 9, **characterised in that** the structures of regions (i) are cluster domains according to Claims 5 to 7.

11. Plaster according to one of Claims 1 to 10, **characterised in that** the cavities of regions (i) which contain the medicament have a thickness which is matched to the medicament depot action to be achieved.

12. Plaster according to one of Claims 1 to 11, **characterised in that** an individual cavity of regions (i) has an average internal diameter of 0.5 to 5.0 mm.

13. Plaster according to one of Claims 1 to 12, **characterised in that** the individual cavities of regions (i) are combined in clusters according to Claims 5 to 7 which have an average internal diameter of 5.0 to 25.0 mm.

14. Plaster according to one of Claims 1 to 13, **characterised in that** the individual cavities of regions (i) containing the medicament are combined in clusters according to Claims 5 to 7, where each cluster domain has 20 to 200 individual trough-like structures of regions (i).

15. Plaster according to one of Claims 1 to 14, **characterised in that** the cavities of regions (ii) are channel-like and have an average internal diameter of 0.3 to 3.0 mm.

16. Plaster according to one of Claims 1 to 15, **characterised in that** the separation between two channel-like cavities of regions (ii) and the separation between two cluster domains of regions (i) is in each case 5 to 25 mm.

17. Plaster according to one of Claims 1 to 16, **characterised in that**, in the case of intersecting channel-like cavities of regions (ii), the connection for a syringe or suction / supply device is installed in the crossing region of two channel structures (i).

18. Plaster according to one of Claims 1 to 17, **characterised in that**, in the case of intersecting channel-like structures of regions (ii), the crossing region is enlarged in the form of a bubble, dome or ball.

19. Plaster according to one of Claims 1 to 18, **characterised in that** it has, on the side facing the wound / skin, a protective film, which is removed before use.

20. Plaster according to one of Claims 1 to 19, **characterised in that** it has, on the side facing the wound / skin, a film or membrane which is permeable for the medicament and is essentially impermeable for proteolytic enzymes from the wound secretion.

21. Plaster according to one of Claims 1 to 20, **characterised in that** the support matrix including its structures of regions (i) and (ii) is made from a polymeric plastic, silicone, a natural elastomer or from a biodegradable material.

22. Plaster according to one of Claims 1 to 21, **characterised in that** the medicament introduced into the cavities of regions (i) is a growth factor.

23. Plaster according to Claim 22, **characterised in that** the growth factor is erythropoietin (EPO) in liquid or lyophilised form or a biologically active derivative or fragment thereof.

24. Use of EPO for the production of a plaster according to one of Claims 1 to 23 for the regenerative healing of damaged skin, mucous membrane or open wounds.

## Revendications

1. Sparadrap pour appliquer sur une peau abîmée, une membrane de muqueuse ou des plaies ouvertes un ou plusieurs composé(s) actif(s) ou médicament(s), comprenant essentiellement une matrice de support qui contient le/les composé(s) actifs ou le/les médicament(s),
**caractérisé en ce que** la matrice de support inclut :
(I) des régions ou structures (i) sous la forme d'une ou de plusieurs cavité(s) jouant le rôle de conteneur pour le médicament, et
(II) des régions ou structures (ii) sous la forme d'une ou de plusieurs cavité(s) jouant le rôle de structures similaires à des canaux qui permettent le logement et le drainage de sécrétions de plaies et/ou l'aération et/ou l'introduction topique d'autres composés actifs et/ou de cellules qui favorisent la cicatrisation de la peau, de la membrane de muqueuse ou d'une plaie ouverte, où
(a) au moins une région selon (i) est adjacente à au moins une région selon (ii),
(b) des régions (i) et (ii) sont fermées sur le côté éloigné de la plaie et lui faisant face,
(c) des régions (i) et (ii) sont ouvertes sur le côté faisant face à la plaie ou sont au moins perméables auxdits médicaments, à ladite sécrétion de plaie, auxdits autres composés/auxdites cellules,
(d) des régions (i) et (ii) formées en tant que cavités sont agencées dans le plan de la surface de sparadrap, et
(e) les cavités de régions (ii) comportent au moins une ouverture ou connexion pour une seringue ou un dispositif d'aspiration/d'application, ce qui permet soit le drainage d'une sécrétion de plaie qui a été collectée dans les cavités grâce à la génération d'une pression réduite générée dans le sparadrap et/soit l'application de n'importe lequel/lesquels desdits autres composés actifs.

2. Sparadrap selon la revendication 1, **caractérisé en ce que** les cavités de régions (ii) sont des canaux qui sont connectés les uns aux autres.

3. Sparadrap selon la revendication 1 ou 2, **caractérisé en ce que** les cavités de régions (i) jouant le rôle de conteneur pour le médicament sont sous la forme de gouttières, où le côté ouvert des gouttières fait face à la plaie, et les gouttières présentent une aire de base rectangulaire, carrée, hexagonale/en forme de nid d'abeille ou ronde.

4. Sparadrap selon l'une des revendications 1 à 3, **caractérisé en ce que** les cavités de régions (i) jouant le rôle de conteneur pour le médicament sont séparées les unes des autres par des bandes compactes ou en forme de canal comprenant le matériau de la matrice de support.

5. Sparadrap selon l'une des revendications 1 à 4, **caractérisé en ce que** les cavités de régions (i) jouant le rôle de conteneur pour le médicament représentent des sous-structures qui sont combinées en groupes qui sont séparés les uns des autres par des bandes compactes ou en forme de canal comprenant le matériau de la matrice de support.

6. Sparadrap selon la revendication 5, **caractérisé en ce que** la forme de l'aire de base de groupe est la même que la forme de l'aire de base des sous-structures de régions (i) ou en est différente.

7. Sparadrap selon la revendication 5 ou 6, **caractérisé en ce qu'**un domaine de groupes de région (i) est adjacent à au moins une cavité de région (ii).

8. Sparadrap selon l'une des revendications 1 à 7, **caractérisé en ce qu'**une cavité de régions (ii) intersecte au moins une autre cavité de régions (ii).

9. Sparadrap selon la revendication 8, **caractérisé en ce que** les cavités de régions (ii) forment un réseau ou une grille rectangulaire, carré(e) ou rhomboïdal(e) dans la matrice de support, entourant des structures de régions (i).

10. Sparadrap selon la revendication 9, **caractérisé en ce que** les structures de régions (i) sont des domaines de groupes selon les revendications 5 à 7.

11. Sparadrap selon l'une des revendications 1 à 10, **caractérisé en ce que** les cavités de régions (i) qui contiennent le médicament présentent une épaisseur qui correspond à l'action de dépôt de médicament à réaliser.

12. Sparadrap selon l'une des revendications 1 à 11, **caractérisé en ce qu'**une cavité individuelle de régions (i) présente un diamètre interne moyen de 0,5 à 5,0 mm.

13. Sparadrap selon l'une des revendications 1 à 12, **caractérisé en ce que** les cavités individuelles de régions (i) sont combinées selon des groupes selon les revendications 5 à 7 qui présentent un diamètre interne moyen de 5,0 à 25,0 mm.

14. Sparadrap selon l'une des revendications 1 à 13, **caractérisé en ce que** les cavités individuelles de régions (i) contenant le médicament sont combinées selon des groupes selon les revendications 5 à 7, où chaque domaine de groupes comporte 20 à 200 structures similaires à des gouttières individuelles de régions (i).

15. Sparadrap selon l'une des revendications 1 à 14, **caractérisé en ce que** les cavités de régions (ii) sont similaires à des canaux et présentent un diamètre interne moyen de 0,3 à 3,0 mm.

16. Sparadrap selon l'une des revendications 1 à 15, **caractérisé en ce que** la séparation entre deux cavités similaires à des canaux de régions (ii) et la séparation entre deux domaines de groupes de régions (i) sont dans chaque cas de 5 à 25 mm.

17. Sparadrap selon l'une des revendications 1 à 16, **caractérisé en ce que**, dans le cas de cavités similaires à des canaux s'intersectant de régions (ii), la connexion pour une seringue ou un dispositif d'aspiration/d'application est installée dans la région de croisement de deux structures similaires à des canaux (i).

18. Sparadrap selon l'une des revendications 1 à 17, **caractérisé en ce que**, dans le cas de structures similaires à des canaux s'intersectant de régions (ii), la région de croisement est agrandie selon la forme d'une bulle, d'un dôme ou d'une balle.

19. Sparadrap selon l'une des revendications 1 à 18, **caractérisé en ce qu'**il comporte, sur le côté faisant face à la plaie/peau, un film de protection, lequel est enlevé avant utilisation.

20. Sparadrap selon l'une des revendications 1 à 19, **caractérisé en ce qu'**il comporte, sur le côté faisant face à la plaie/peau, un film ou une membrane qui est perméable au médicament et est essentiellement imperméable à des enzymes protéolytiques provenant de la sécrétion de plaie.

21. Sparadrap selon l'une des revendications 1 à 20, **caractérisé en ce que** la matrice de support y compris ses structures de régions (i) et (ii) est réalisée à partir d'une matière plastique polymérique, d'un silicone, d'un élastomère naturel ou à partir d'un matériau biodégradable.

22. Sparadrap selon l'une des revendications 1 à 21, **caractérisé en ce que** le médicament introduit à l'intérieur des cavités de régions (i) est un facteur de croissance.

23. Sparadrap selon la revendication 22, **caractérisé en ce que** le facteur de croissance est de l'érythropoïétine (EPO) sous forme liquide ou lyophilisée ou un dérivé biologiquement actif ou un fragment afférent.

24. Utilisation d'EPO pour la production d'un sparadrap selon l'une des revendications 1 à 23 pour la cicatrisation régénérative d'une peau abîmée, d'une membrane des muqueuses ou de plaies ouvertes.
